(19) Europäisches Patentamt
European Patent Office
Office européen des brevets

(11) **EP 0 995 993 B1**

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(51) Int Cl.7: **G01N 33/52**

(21) Anmeldenummer: **99120059.3**

(22) Anmeldetag: **19.10.1999**

(54) **Funktionelle Auflage für flexible Objekte, insbesondere für diagnostische Teststreifen**

Functional coating for flexible objects, particularly for diagnostic test strips

Couche fonctionelle pour les objets flexibles, en particulier pour les bandes d'essai diagnostiques

(84) Benannte Vertragsstaaten:
**DE ES FR GB IT**

(30) Priorität: **23.10.1998 DE 19849024**

(43) Veröffentlichungstag der Anmeldung:
**26.04.2000 Patentblatt 2000/17**

(73) Patentinhaber: **Roche Diagnostics GmbH**
**68305 Mannheim (DE)**

(72) Erfinder:
• **Knappe, Wolfgang, Dr.**
**67061 Ludwigshafen (DE)**
• **Leininger, Helmut**
**68259 Mannheim (DE)**
• **Steinbrueck, Ralf**
**68305 Mannheim (DE)**
• **Wittmann, Franz**
**68766 Hockenheim (DE)**

(56) Entgegenhaltungen:
EP-A- 0 291 843    EP-A- 0 821 233
DE-A- 4 015 589    DE-B- 2 118 455
GB-A- 1 276 301    GB-A- 2 291 354
US-A- 4 871 600

**Beschreibung**

Die vorliegende Erfindung betrifft Konstruktionen umfassend ein flächenförmiges, flexibles Objekt und nicht vollflächig fixierte funktionelle Auflagen, die so auf dem flexiblen Objekt befestigt sind, daß sie bei einer Biegung des Objekts nach der Seite, auf der die Auflage liegt, keine Falte bilden,
sowie funktionelle Auflagen für die Abdeckung analytsensitiver Felder von Teststreifen, die eine gleichmäßige Verteilung aufgebrachter analythaltiger Proben auch dann gewährleistet, wenn der Teststreifen mehr als ein einzelnes Nachweisfeld aufweist. Die Erfindung betrifft ferner Teststreifen, die mit derartigen Auflagen ausgerüstet sind und deren Verwendung zur Bestimmung diagnostisch relevanter Analyte.

Funktionelle Auflagen im Sinne dieser Erfindungsbeschreibung sind solche, die eine Funktion ausüben, ohne die der bestimmungsgemäße Gebrauch des Gegenstands, dem sie angehören, unmöglich wäre.

Zur qualitativen oder quantitativen analytischen Bestimmung von Bestandteilen von Körperflüssigkeiten, insbesondere von Blut, werden oft sogenannte trägergebundene Tests verwendet. Bei diesen liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und darauf angebrachten Nachweisschichten als Testfelder bestehen. Es sind jedoch auch Testträger bekannt, die als quadratische oder rechteckige Plättchen gestaltet sind. In der folgenden Beschreibung soll die Bezeichnung "Teststreifen" auch Testträger umfassen, die nicht die Streifenform aufweisen.

Testträger der eingangs bezeichneten Art sind beispielsweise aus der deutschen Patentschrift 21 18 455 bekannt. Dort werden diagnostische Testträger zum Nachweis von Analyten in Flüssigkeiten beschrieben, die aus einer Tragschicht und mindestens einer die Nachweisreagenzien enthaltenden Nachweisschicht, deren nicht auf der Tragschicht anliegende Oberfläche mit einer Deckschicht versehen ist, bestehen. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu vermeiden. Zum Nachweis eines Analyts in einer Flüssigkeit wird ein solcher diagnostischer Testträger in eine entsprechende Flüssigkeit, vorzugsweise Urin, eingetaucht. Die Nachweisschicht kommt so mit einem sehr großen Überschuß an Flüssigkeit in Kontakt, der nicht von dem Testträger aufgenommen werden kann. Je nach der Dauer des Kontaktes der Nachweisschicht mit der zu untersuchenden Flüssigkeit können jedoch unterschiedliche Farbintensitäten beobachtet werden.

In der Regel werden um so positivere Resultate erhalten, je länger die Kontaktzeit ist. Bei einem großen Probenüberschuß ist daher eine korrekte quantitative Analytbestimmung auf diese Weise nicht möglich.

Andererseits ist ein für eine Testträgerkonstruktion geringes Probenvolumen eine häufige Ursache für falsche Meßwerte beim Diabetes-Monitoring, das heißt, der regelmäßigen Kontrolle des Blutes Diabeteskranker auf den Gehalt an Glucose.

Testträger mit möglichst geringem Volumenbedarf sind deshalb das Ziel vielfältiger derzeitiger Entwicklungen.

Diagnostische Testträger in Form von Teststreifen, die einen deutlichen Fortschritt in Bezug auf Reproduzierbarkeit der Testergebnisse selbst bei Applikation unterschiedlicher Probenvolumina und in Bezug auf hygienische Handhabbarkeit bieten, sind aus der EP-A-0 821 233 bekannt.

Sie enthalten eine Tragschicht mit einer darauf angeordneten, zur Bestimmung von Analyt in flüssiger Probe erforderliche Reagenzien enthaltenden Nachweisschicht und eine die Nachweisschicht überdeckende Auflage aus einem Netzwerk, die größer als die Nachweisschicht ist und die beiderseits der Nachweisschicht auf der Tragschicht, vorzugsweise über einen Abstandshalter, befestigt ist, auf der Nachweisschicht dagegen ohne Befestigung unmittelbar, d.h. diese ohne Abstand im wesentlichen vollflächig berührend, aufliegt. Das als Auflage eingesetzte Netzwerk soll hydrophil aber alleine nicht kapillaraktiv sein. Über den über die Nachweisschicht hinausragenden Bereichen der Auflage, ist eine inerte Abdeckung aus probenundurchlässigem Material so angeordnet, daß eine Probenauftragsstelle auf dem die Nachweisschicht überdeckenden Bereich des Netzwerkes frei bleibt.

Das die Nachweisschicht überdeckende Netzwerk besteht bei dieser Konstruktion vorzugsweise aus einem grobtitrigen, relativ grobmaschigen Monofilgewebe mit einer ausreichend großen Maschenweite, damit Flüssigkeit durch das Netz auf die Nachweisschicht gelangt (Seite 3, Zeile 12). Im Beispiel dieser Anmeldung wird ein Monofilgewebe mit einer Maschenweite von 280 µm eingesetzt. Ihm kommt eine wesentliche Funktion zu: Es leitet auf seine Oberfläche aufgebrachte Probenflüssigkeit schnell auf die darunterliegende Nachweisschicht weiter. Wenn die Nachweisschicht gesättigt ist, wird ein eventuell vorhandener Probenüberschuß in die über die Nachweisschicht hinausgehenden Randbereiche des Netzwerks abgeleitet. Auf diese Weise werden geringe Probenmengen der Nachweisschicht vollständig zur Verfügung gestellt, die längere Einwirkung eines Probenüberschusses, die zu Fehl-Positiv-Ergebnissen führen kann, aber vermieden.

In dieser Druckschrift ist auch eine Ausführungsform beschrieben, die zwei oder mehrere nebeneinander angeordnete Nachweisschichten aufweist, die zur Messung des gleichen oder verschiedener Analyte dienen sollen.

Die Verwendung von Testträgern in dieser Ausführungsform bietet natürlich auf den ersten Blick verlockende Vorteile bezüglich Arbeits- und Kostenersparnis, da es mit ihnen möglich sein sollte, mit einem einzigen Probenauftrag zwei oder gar mehrere Messungen auszuführen. In der Praxis ergeben sich aber beim Versuch, diese Ausführungsform einzusetzen, verschiedene Schwierigkeiten. Eine wesentliche Forderung, daß das für die Messung erforderliche Probenmindestvolumen 6 µl nicht überschreiten sollte, läßt sich mit einer solchen Testträgerkonstruktion, bei der die Nachweisfelder eine Fläche von jeweils etwa 20 bis 40 mm² haben und das besagte Netzwerk über beide Nachweisfelder läuft, nicht erfüllen. Es zeigt sich nämlich, daß hierbei nur das Testfeld benetzt wird, über dem die Probe aufgetragen wurde. Eine Benetzung des zweiten Testfeldes findet selbst bei Aufgabe von Probenvolumina bis zu 30 µl nicht statt. Auch der Versuch, eine Benetzung beider Testfelder dadurch zu erreichen, daß die Felder auf dem Träger ohne Zwischenraum unmitelbar nebeneinander plaziert werden, führt nicht zum Erfolg. Es zeigt sich, daß bereits der auch in diesem Fall immer noch vorhandene winzige Abstand der Testfelder von 5 bis 10 µm das Blut daran hindert, von einem auf das andere Feld "überzuspringen".

Die Benetzung beider Testfelder findet nur dann statt, wenn die Probe genau über dem Spalt zwischen den beiden Testfeldern aufgetragen wird. Eine so exakte Probenaufgabe ist in der Praxis jedoch nur schwer zu bewerkstelligen. Eine Benetzung beider Testfelder läßt sich zwar erreichen, wenn man in der inerten Abdeckung über dem Netzwerk einen nur ca. 2 bis 3 mm breiten, über dem Spalt zwischen den Meßfeldern angeordneten Auftragsschlitz frei läßt, jedoch benötigt man bei diesem Aufbau zur vollständigen Benetzung der Testfelder ein Mindest-Probenvolumen von über 15 µl.

Es wurde nun gefunden, daß sich eine Reduzierung des für die Messung erforderlichen Probenvolumens auf ca. 4 µl erzielen läßt, wenn man anstelle des grobtitrigen, grobmaschigen Netzwerks eine funktionelle Auflage mit abgestimmtem Flüssigkeitsverteilungsvermögen (Spreitvermögen), vorzugsweise aus einem dünnen Textilmaterial aus feintitrigen Monofilamenten oder entsprechenden Multifilamentgarnen, in die Testkonstruktion einbaut.

Dünne textile Spreitgewebe, Spreitgewirke oder Spreitvliese, die auf die Erfordernisse des Einzelfalls abgestimmte Durchlässigkeit, Leitfähigkeit und Kapillaraktivität und in der Regel ein geringes Speichervermögen aufweisen, sind demnach für anspruchsvollere Spreitaufgaben, insbesondere für das Spreiten geringer Flüssigkeitsvolumina über zwei oder mehrere verschiedene Nachweisfelder erheblich besser geeignet, als die in der EP-A-0 821 233 dafür empfohlenen grobtitrigen Netzwerke. Es wurde ferner gefunden, daß es für die einwandfreie Spreitung des Analyts erforderlich ist, daß die Spreitauflage mit der Unterlage, über die gespreitet werden soll in gutem Kontakt steht.

Aus dieser Notwendigkeit ergibt sich nun bei der Verwendung dünner funktioneller Auflagen, deren einwandfreie Funktion einen guten Kontakt zur Unterlage voraussetzt, ein Problem wenn die Teststreifen zur Auswertung in ein Meßgerät - wie beispielsweise das bekannte GLUCOTREND®-Gerät - eingebracht werden, in dem sie zum Zwecke der exakten Positionierung durch eine Biegung unter mechanische Spannung gesetzt werden. Erfolgt die Biegung nach der Seite, auf der sich die erfindungsgemäße funktionelle Auflage befindet, so neigt diese dazu, ausgerechnet im Bereich der Nachweisschicht, wo die Auflage unfixiert frei aufliegt, eine Falte zu bilden, d.h. sich von der Nachweisschicht abzuheben, wodurch das gleichmäßige Füllen der Nachweisschicht erheblich beeinträchtigt oder gar unmöglich wird. Insbesondere bei Teststreifen mit zwei nebeneinander liegenden Testfeldern wird die gleichmäßige Versorgung beider Testfelder beim Auftragen der Probe verhindert.

Zur Veranschaulichung der folgenden Ausführungen dienen die Figuren 1 bis 14.

Die Figur 1 zeigt schematisch einen Schnitt durch einen Abschnitt aus einem Teststreifen mit einer dünnen funktionellen Auflage.

Die Figuren 2 und 3 zeigen schematisch einen Schnitt durch einen Abschnitt eines mit einer dünnen funktionellen Auflage versehenen Teststreifen in gebogener Form.

Die Figur 4 zeigt schematisch Schnitt durch einen Abschnitt aus einem Teststreifen mit einer erfindungsgemäß fixierten funktionellen Auflage.

Die Figur 5 zeigt den in Figur 4 dargestellten Schnitt des Objekts mit der erfindungsgemäß fixierten funktionellen Auflage in gebogener Form.

Die Figur 6 zeigt schematisch einen Schnitt durch einen Abschnitt aus einem Teststreifen mit einer erfindungsgemäß fixierten funktionellen Auflage.

Die Figur 7 zeigt den in Figur 6 dargestellten Schnitt des Aufbaus mit den erfindungsgemäß fixierten Auflageelementen in gebogener Form.

Die Figur 8 zeigt eine perspektivische Aufsicht, die Figur 9 einen Schnitt längs der Schnittlinie A-A', die Figur 10 eine Aufsicht auf die Unterseite einer Ausführungsform eines erfindungsgemäßen Teststreifens.

Die Figur 11 zeigt eine Aufsicht auf einen Abschnitt eines Teststreifens, Figur 12 eine Schnittdarstellung längs der Schnittlinie A-A'dieses Objekts.

Die Figur 13 zeigt eine Aufsicht auf einen Abschnitt eines Teststreifens mit einer Folge von Nachweisfeldern die von einer Reihe sich schindelartig überlappender erfindungsgemäßer Auflageelemente überdeckt ist, Figur 14 eine Schnittdarstellung längs der Schnittlinie A-A' dieses Objekts.

Die in den Figuren verwendeten Bezugszeichen haben folgende Bedeutung:

| | |
|---|---|
| 1: | Teststreifen |
| 2: | Flexibler Träger |
| 3 bis 3g: | Nachweisschichten |
| 4 bis 4c: | Abstandshalter |
| 5 und 5a: | Adhäsionsschichten |
| 6: | Erfindungsgemäße funktionelle Auflage |
| 6a bis 6f: | Erfindungsgemäße Auflageelemente |
| 7: | Befestigungsbereich |
| 8 und 8a: | Verschiebbarer Bereich |
| 9: | Beliebige Auflage |
| 10 und 10a: | Schutzabdeckung |
| 11: | Auftragsbereich |
| 12 und 12a: | Markierung der Grenzen der Nachweisfelder |
| 13: | Markierung der Einschubrichtung |
| 14: | Positionierungsbohrung |
| 15 und 16: | Beobachtungs- und Meßöffnungen |
| 17 bis 17d: | Befestigungspunkte für Auflageelemente |
| 18 bis 18d: | Befestigungspunkte für Auflageelemente. |

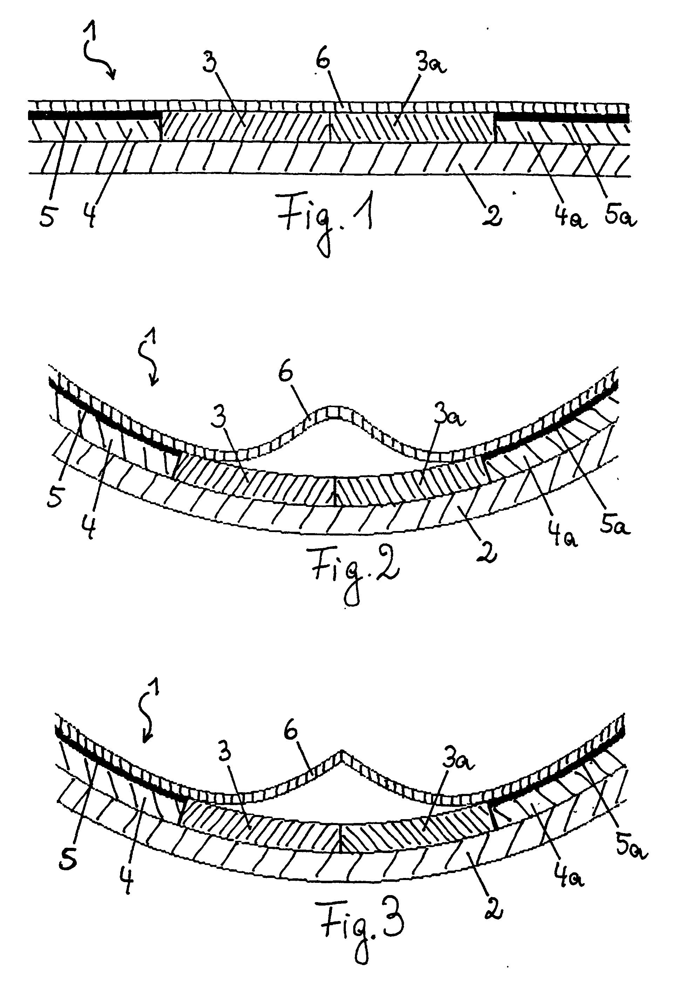

Die Figur 1 zeigt schematisch, zur klaren Veranschaulichung in nicht maßstabsgerechter Darstellung, einen Schnitt durch einen Abschnitt aus einem Teststreifen (1), der auf einem Träger (2) zwei unmittelbar nebeneinander angeordnete Nachweisschichten (3 und 3a) aufweist, an die sich rechts und links Abstandshalter (4 und 4a) anschließen. Auf den Abstandshaltern ist jeweils eine Adhäsionsschicht (5 und 5a) aufgebracht, durch welche die die Nachweisschicht lose aufliegend überspannende dünne funktionelle Auflage (6) beiderseits der Nachweisschichten fixiert wird. Die Abstandshalter haben zusammen mit der Adhäsionsschicht zweckmäßigerweise etwa die gleiche Dicke wie die Nachweisschichten. Die zwischen der zur Auflage gerichteten Fläche der Adhäsionsschicht und den Nachweisschichten eventuell vorhandene Stufe sollte so gering sein, daß die vollflächige Berührung der Auflage mit den Nachweisschichten nicht in relevantem Umfang beeinträchtigt wird.
Die selbstverständlich vorhandene Befestigung der Nachweisschichten und der Abstandshalter auf dem Träger, die ebenfalls über Adhäsionsschichten erfolgen kann, ist in der Figur nicht dargestellt.

Die Figuren 2 und 3 zeigen schematisch, zur klareren Veranschaulichung in nicht maßstabsgerechter Darstellung und mit übertriebener Biegung, einen Querschnitt durch einen Abschnitt eines mit einer dünnen funktionellen Auflage versehenen Teststreifens (1), wie er bereits in Fig 1 veranschaulicht wurde, der nach der Seite, auf der die Auflage angebracht ist, gebogen wurde.

Der Teststreifen (1) weist auf einem Träger (2) zwei unmittelbar nebeneinander angeordnete Nachweisschichten (3 und 3a) auf, an die sich rechts und links Abstandshalter (4 und 4a) anschließen. Auf den Abstandshaltern ist jeweils eine Adhäsionsschicht (5 und 5a) aufgebracht, durch welche die Auflage (6) beiderseits der Nachweisschichten fixiert wird. Man erkennt, daß sich durch die Biegung die Auflage in einem großen Bereich von den Nachweisschichten abgehoben hat und eine Falte bildet, deren Form naturgemäß u.a. von der Biegesteifheit, der Konstruktionskompressibilität und Homogenität des Auflagematerials abhängt.

Es stellte sich daher die Aufgabe, eine Konstruktion zu finden, bei der trotz Verwendung einer Auflage aus einem relativ dünnen Auflagenmaterial, das im Prinzip in der Lage ist, von einem Auftragspunkt aus gegebenenfalls mehrere Testfelder gleichmäßig mit Probenmaterial zu versorgen, die Bildung von Falten im Bereich der Biegung des Teststreifens vermieden wird. Verallgemeinert bedeutet dies, eine Konstruktion zu finden, um auf einem flexiblen Träger eine nicht vollflächig fixierte Auflage so anzubringen, daß bei einer Biegung des Verbunds nach der Seite, auf der sich die Auflage befindet, in letzterer auch im nicht fixierten Flächenbereich keine Falte entsteht.

In flächenförmigen, nicht sehr dünnen, flexiblen Objekten, die aus einem Verbund mehrerer Schichten bestehen, werden beim Verbiegen die auf der Innenseite der Krümmung liegenden Schichten gestaucht, die auf der Außenseite liegenden Schichten gedehnt. Ist bei solchen Schichtsystemen die auf der Innenseite der Krümmung liegende Schicht nicht vollflächig, sondern nur an beabstandeten Punkten mit der oder den anderen Schichten verbunden, so neigt diese Schicht an den nicht fest mit der Oberfläche des Objekts verbundenen Flächenbereichen dazu, durch Faltenbildung dem Stauchungszwang auszuweichen. Die Faltenbildung stellt somit eine Auswirkung des in der Natur stets wirksamen "Prinzips des kleinsten Zwanges" dar, das es mit der gesuchten Konstruktion zu überwinden galt.

Die Lösung dieser Aufgabe gelingt erfindungsgemäß mittels einer Konstruktion umfassend ein flächenförmiges flexibles Objekt und einer flächenförmigen funktionellen Auflage für zumindest einen Bereich des flächenförmigen, flexiblen Objekts, die beim Biegen des Verbunds bestehend aus flexiblem Objekt und Auflage nach der Seite, auf der sich die Auflage befindet keine Falte bildet, die dadurch gekennzeichnet ist, daß sie aus einem oder mehreren flächenförmigen Auflageelementen besteht, die auf dem flexiblen Objekt so befestigt sind, daß zumindest ein Teil ihrer Fläche gegenüber der von diesem Teil abgedeckten Objektfläche in Richtung der beim Biegen des Objekts erzeugten Krümmung überlappend angeordnet ist, damit die Fläche frei verschiebbar ist.

Die Befestigung eines Auflageelements der erfindungsgemäßen Auflage auf dem flexiblen Objekt erfolgt durch mindestens einen, vorzugsweise mindestens zwei, Befestigungspunkte, die in einem zusammenhängenden Flächenbereich (Befestigungsbereich) des Auflageelements liegen, der sich zwischen den in Krümmungsrichtung liegenden Kanten des Auflageelements erstreckt und dessen Abgrenzung vom frei verschiebbaren Teil des Auflageelements im wesentlichen geradlinig und quer zur Krümmungsrichtung verläuft.

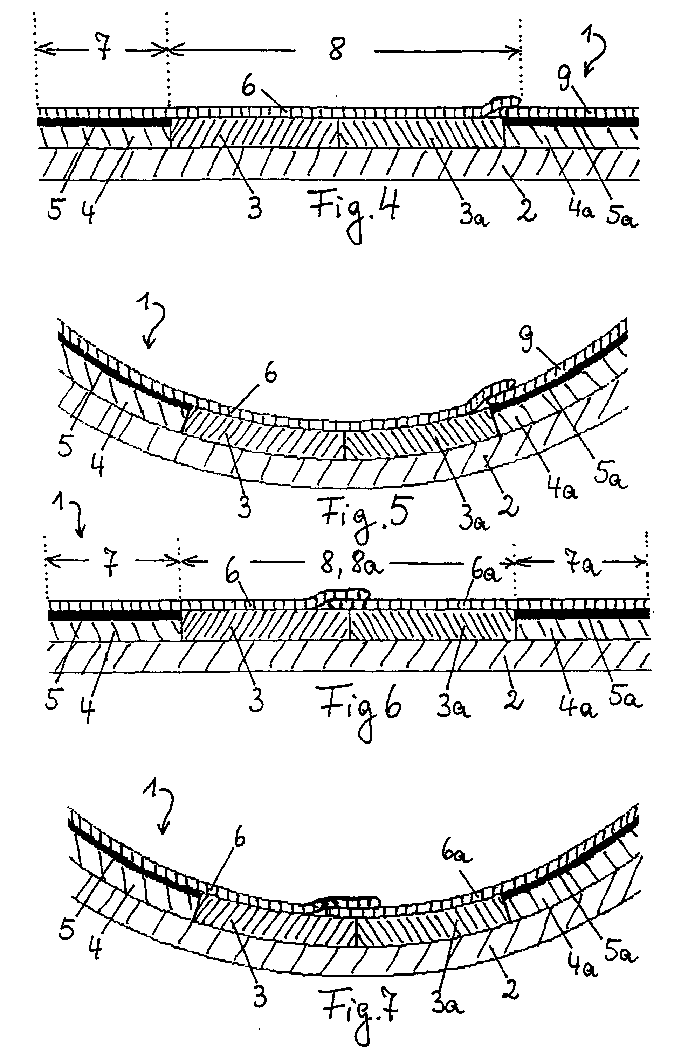

Die Figur 4 zeigt schematisch, zur klareren Veranschaulichung in nicht maßstabsgerechter Darstellung, einen Querschnitt durch einen Abschnitt aus einem Objekt mit einer erfindungsgemäß fixierten funktionellen Auflage, hier zur Konkretisierung wieder in Form eines Teststreifens (1), der auf einem Träger (2) zwei unmittelbar nebeneinander angeordnete Nachweisschichten (3 und 3a) aufweist, an die sich links ein Abstandshalter (4) anschließt. Auf dem Abstandshalter ist eine Adhäsionsschicht (5) aufgebracht, durch welche die erfindungsgemäße Auflage (6) erfindungsgemäß einseitig in ihren Befestigungsbereich (7) fixiert wird. Der in Krümmungsrichtung frei verschiebbare Abdeck-Bereich (8), der Auflage (6) überdeckt die Nachweisschicht lose aufliegend und überlappt an seinem freien Ende mit einer beliebigen Auflage (9), die ihrerseits über einen Abstandshalter (4a) mit Adhäsionsschicht (5a) vollflächig auf dem Träger fixiert ist. Die Dicke des Aufbaus aus Auflage (9), Abstandshalter (4a) und Adhäsionsschicht (5a) entspricht in dieser Darstellung der Dicke der Nachweisschichten. Die Abstandshalter (4) haben zusammen mit der Adhäsionsschicht (5) zweckmäßigerweise etwa die gleiche Dicke wie die Nachweisschichten.

Die Figur 5 zeigt den in Figur 4 dargestellten Querschnitt des Objekts mit der erfindungsgemäß fixierten funktionellen Auflage in gebogener Form. Man erkennt, daß hier die Auflage (6) trotz der Biegung keine Falte bildet, sondern in ihrem gesamten Überdeckungsbereich auf den Nachweisschichten (3) und (3a) lückenlos aufliegt. Dafür hat sich durch Verschiebung des verschiebbaren Endes der erfindungsgemäßen Auflage (6) in Krümmungsrichtung ihre Überlappung mit der Auflage (9) vergrößert.

Jedes Auflageelement ist somit in einen Befestigungsbereich und in einen auf dem Objekt verschiebbaren Abdeckbereich unterteilt. Die Grenze zwischen den Bereichen erstreckt sich im wesentlichen quer zur Krümmungsrichtung des flexiblen Objekts.

Eine mögliche Einteilung eines rechteckigen Auflageelements in einen verschiebbaren Bereich und einen Befestigungsbereich besteht darin, daß der Befestigungsbereich das eine Ende, der verschiebbare Bereich das andere Ende des Auflageelements umfaßt, wobei die Grenze zwischen beiden Bereichen parallel zu einer Kante quer zur Krümmumgsrichtung des flexiblen Objekts verläuft.

Natürlich kann die Befestigung des Auflageelements auch in einem streifenförmigen Bereich in der Mitte des Element erfolgen, sodaß beide Enden auf dem Objekt frei verschiebbar sind.

Für das Größenverhältnis der Flächen von Befestigungsbereich und verschiebbaren Bereich gibt es im Prinzip keine Einschränkung. Die Flächengrößen richten sich nach den speziellen Erfordernissen des Einzelfalls. Die Größe des Befestigungsbereichs muß ausreichen, eine sichere Befestigung zu gewährleisten, sie kann aber auch erheblich vergrößert werden, wenn dies für besondere Funktionen erwünscht ist. Die Fläche des verschiebbaren Auflagebereichs, insbesondere ihre Erstreckung in Richtung der Krümmung, richtet sich nach der Steife des Auflagematerials, der zu erwartenden Stärke der Krümmung und natürlich der Größe der von der Auflage zu überdeckenden Fläche. Bei geringer zu erwartender Krümmung kann die Erstreckung in Krümmungsrichtung größer sein als bei stärkeren Krümmungen. Die für einen geplanten Anwendungsfall zweckmäßige Länge des verschiebbaren Auflagebereichs kann man am einfachsten durch einige wenige systematische Versuche ermitteln.

Eine einfache, insbesondere bei Einsatz nur eines Auflageelements, mögliche und zweckmäßige Befestigungsart besteht darin, daß - wie in den Figuren 4 und 5 dargestellt - das Auflageelement im Befestigungsbereich vollflächig auf dem flexiblen Objekt fixiert ist.

Um trotz der unterschiedlichen Verschiebung des verschiebbaren Teils des Auflageelements bei verschiedenen Krümmungen stets eine vollständige Überdeckung des Objekts oder des zu überdeckenden Bereichs des Objekts zu erreichen, ist es zweckmäßig, daß die Auflage - wie in den Figuren 4 und 5 dargestellt - an ihrem verschiebbaren Ende mit einer Auflage gleicher oder beliebig anderer Befestigungsart oder mit irgendeiner anderen Abdeckung des zu überdeckenden Bereichs des Objekts überlappt.

Diese Überlappung kann, wenn zwei Auflageelemente für die Abdeckung des abzudeckenden Bereichs des flexiblen Objekts erforderlich sind und ausreichen, auf elegante Weise erreicht werden, indem man die beiden Auflageelemente so auf dem Objekt fixiert, daß deren verschiebbare Bereiche gegeneinander gerichtet sind und sich überlappen.

Die Figur 6 veranschaulicht diesen Aufbau: Sie zeigt schematisch, zur klareren Veranschaulichung in nicht maßstabsgerechter Darstellung, einen Querschnitt durch einen Abschnitt aus einem Objekt mit einer erfindungsgemäß fixierten Auflage, hier zur Konkretisierung in Form eines Teststreifens (1), der auf einem Träger (2) zwei unmittelbar nebeneinander angeordnete Nachweisschichten (3 und 3a) aufweist, an die sich links und rechts Abstandshalter (4 und 4a) anschließen. Auf den Abstandshaltern sind Adhäsionsschichten (5 und 5a) aufgebracht, durch welche die erfindungsgemäßen Auflageelemente (6 und 6a) jeweils erfindungsgemäß einseitig in ihren Befestigungsbereichen (7 und 7a) fixiert werden. Die in Krümmungsrichtung frei verschiebbaren Bereiche (8 und 8a), der Auflagen überdecken die Nachweisschicht lose aufliegend und überlappen an ihren freien Enden miteinander. Die Abstandshalter haben zusammen mit der Adhäsionsschicht zweckmäßigerweise etwa die gleiche Dicke wie die Nachweisschichten.

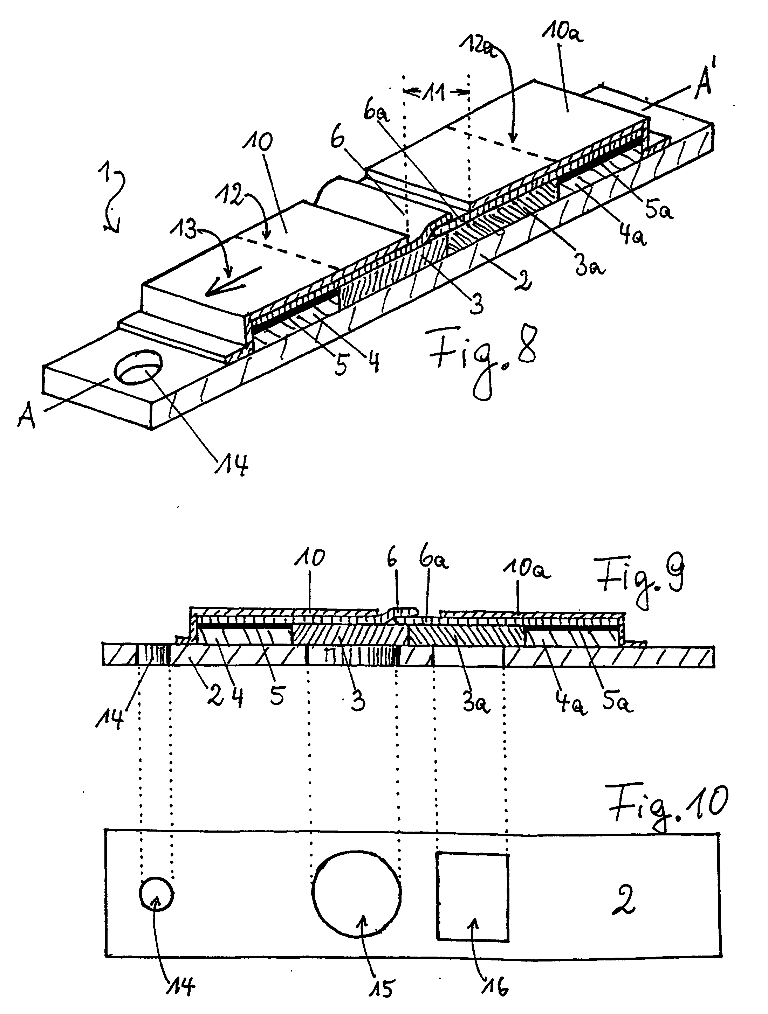

Die Figur 7 zeigt den in Figur 6 dargestellten Querschnitt des Aufbaus mit den erfindungsgemäß fixierten Auflageelementen in gebogener Form. Man erkennt, daß hier die Auflageelemente (6 und 6a) trotz der Biegung keine Falte bilden, sondern in ihrem gesamten Überdeckungsbereich auf den Nachweisschichten (3) und (3a) praktisch lückenlos aufliegt. Die sehr geringe Abhebung des Auflageelements (6) unmittelbar vor der Überlappung mit dem Auflageelement (6a) verursacht keine Störung der Probenverteilung auf die Nachweisschichten.

In der Praxis hat es sich gezeigt, daß es ausreichend ist, wenn die Auflageelemente auf einer Länge von 0,5 bis 2,5 mm überlappen.

Obwohl der Erfindung zunächst nur die Aufgabe zugrundelag, relativ dünne Spreitauflagen, die für die Auswertung geringer Probenvolumina geeignet sind, auf Teststreifen so zu befestigen, daß sie mit der (den) Nachweisschicht(en) auch dann in vollflächigem Kontakt bleiben, wenn der Teststreifen gebogen wird, ist die Erfindung nicht auf die Lösung dieser speziellen Aufgabe beschränkt. Vielmehr empfielt sich die erfindungsgemäße Art der Befestigung für alle funktionellen Auflagen, deren Funktion im Gebrauch auf einem losen, aber möglichst lückenlosen, d.h. vollflächigen Kontakt mit einer flexiblen Unterlage beruht, sofern im Gebrauch eine Biegung der Unterlage eintreten kann.

Gerade bei Teststreifen, deren einwandfreier Funktion eine besondere Bedeutung zukommt, die auch unter verschiedenen Einsatzbedingungen und bei der Anwendung durch mehr oder weniger geübte Laien gewährleistet sein muß, stellt die erfindungsgemäße Befestigungsart einen sehr wertvollen Beitrag zur Anwendungssicherheit dar. Ihr Einsatz auf Teststreifen ist daher ebenfalls ein Gegenstand der Erfindung und oben auch zur beispielhaften Veranschaulichung der Erfindung in den Figuren dargestellt worden.

Besonders wertvolle Dienste leistet die Erfindung bei der Herstellung von Teststreifen, bei denen eine sehr geringe Blutmenge über größere, gegebenenfalls auch mehrere Testflächen gleichmäßig verteilt werden soll. Für solche Teststreifen wird zum Beispiel gefordert, daß ca. 5µl Blut über eine gegebenenfalls unterteilte Testfläche von 30 bis 40 mm$^2$ verteilt werden sollen.

Vorzugsweise werden in solchen Testträgern funktionelle Auflagen zur Überdeckung von zumindest Teilflächen, insbesondere die Nachweisschichten, von Testträgern eingesetzt, die dadurch gekennzeichnet sind, daß sie aus einem offenporigen Flächengebilde aus einem Textilmaterial bestehen, das selbst nicht kapillaraktiv aber flüssigkeitsdurchlässig ist, und dessen Konstruktion und Material und/oder Hydrophilierungsausrüstung so gewählt wird, daß es, auf einer Unterlage aufliegend, 10 µl Wasser auf eine Fläche von mehr als 300 mm$^2$ spreitet.

Textile Flächengebilde, die diese Forderung erfüllen, finden sich unter handelsüblichen Materialien. Die grobe Auswahl geeigneter Textilmaterialien kann grundsätzlich nach der Devise "möglichst dünn bei möglichst kleinem Flächengewicht" erfolgen. Zur Feinauswahl geeigneter Materialien kann der folgende Spreit-Test dienen:

10 mm breite und mindestens 100 mm lange Streifen des zu prüfenden, gegebenenfalls hydrophil ausgerüsteten, Textilmaterials werden auf die mattierte Seite einer Polycarbonatfolie aufgelegt. Dann trägt man in der Mitte des Streifens 10 µl Wasser punktförmig auf und beobachtet seine Ausbreitung. Bei einem geeigneten Material soll der Ausbreitungsprozeß innerhalb von 5 bis 10 Sekunden abgeschlossen sein, und die benetzte Fläche mindestens 30x10 mm betragen.

Mit diesem Test lassen sich beispielsweise die gravierenden Unterschiede zwischen dem gemäß EP-A-0 821 233 als Spreitmaterial einzusetzenden Polyestergewebe PE 280 HC und erfindungsgemäß einzusetzenden Textilmaterialien, z.B. Polyestergewebe PE 38 HC oder Viledon-Vliesstoff FO 2451/121 wie folgt demonstrieren:

10 mm breite Streifen der zu testenden Textilmaterialien werden mit der gleichen Menge (ca. 0,25 Gew.-%, bezogen auf Materialgewicht vor dem Imprägnieren) Natrium-N-oleoyl-sarcosinat imprägniert und getrocknet. Darm werden die imprägnierten Textilstreifen auf die mattierte Seite einer Polycarbonatfolie aufgelegt. Trägt man nun 10 µl Wasser punktförmig auf, so spreitet das Wasser in 5 bis 10 Sekunden über die in der folgenden Tabelle angegebenen Flächen und kommt dann zum Stillstand:

| Gemäß | Material | Fläche |
|---|---|---|
| EP-A-0821233 | PE 280 HC | 8x10 mm |
| Erfindung | PE 38 HC | 40x10 mm |
| Erfindung | Viledon FO 2451/121 | 50x10 mm |

Als Textilmaterialien, die für die Spreitung geringer Blutmengen besonders geeignet sind, haben sich Gewebe, Gewirke oder Vliese mit einer Dicke von 20 bis 200 µm, vorzugsweise 30 bis 100 µm und/oder einem Porenvolumen von 30 bis 85, vorzugsweise von 40 bis 75 %. erwiesen. Das Flächengewicht dieser für die Spreitung geringer Probenvolumina besonders geeigneter Materialien beträgt 10 bis 200, vorzugsweise 10 bis 50 g/m$^2$.

Besonders bevorzugt sind in diesem Rahmen die Materialien mit Dicken unter 100 µm insbesondere unter 50 µm und Flächengewichten von unter 50, vorzugsweise unter 25 g/m$^2$.

Besonders bevorzugte Flächengebilde mit den genannten Dimensionen sind Gewebe und Vliese. Von den Geweben sind solche bevorzugt, die eine Maschenweite unter 200 µm, vorzugsweise von 20 bis 150 µm, insbesondere von 20 bis 60 µm, aufweisen, schiebefest sind und aus Monofilamenten mit Titern im Bereich von 2 bis 20 dtex, vorzugsweise von 4 bis 15 dtex, in Leinwandbindung hergestellt sind. Die Gewebe können eine nach dem Weben aufgebrachte Schiebefestausrüstung aufweisen. Vorzugsweise wird die Verschiebefestigkeit der Gewebe jedoch dadurch erreicht, daß den Monofilamenten im Zuge der Gewebeherstellung eine wellenförmige Verformung aufgeprägt wird und/oder daß Kette- und Schußfilamente an den Kreuzungspunkten leicht miteinander verschmolzen werden, wodurch sie gegen Verschieben gesichert werden.

Erfindungsgemäß eingesetzte Vliese sind Wirrvliese, vorzugsweise gebondete Spinnvliese. Sie bestehen aus endlosen Monofilamenten, deren Titer im Bereich von 0,5 bis 2,5 dtex, vorzugsweise von 0,8 bis 2,0 dtex liegt. Das Bonden kann durch Behandlung der Vliese mit Bondierungsmitteln erfolgen; bevorzugt ist jedoch das "autogene" Bonden, bei dem die Filamente an ihren Kreuzungspunkten miteinander leicht verschmolzen werden. Die Poren derartiger Vliese weisen naturgemäß erhebliche Größenunterschiede auf. Überraschenderweise sind Vliese mit den oben angegebenen Merkmalen trotz großer Streubreite der Porengröße für den erfindungsgemäßen Einsatz hervorragend geeignet.

Die geringe oder nicht vorhandene Kapillaraktivität des Flächengebildes zeigt sich darin, daß es eine Steighöhe von Wasser bei 25°C von unter 2 mm in 10 sec. aufweist.

Als Fasermaterialien für die erfindungsgemäß einzusetzenden Textilmaterialien kommen sowohl natürliche Fasern wie Zellulose oder Eiweisfasern, halbsynthetische Fasern wie Acetat- oder Viskoseseide oder vollsynthetische Fasern wie Polyester-, Polyamid-, Polyurethan- oder Polyacrylnitril- Polyethylen oder Polypropylenfasem oder Mischungen aus den genannten Fasertypen in Betracht.

Bevorzugte Zellulosefasern sind Baumwollefasern, bevorzugte Eiweisfasern sind Wolle und Naturseide. Geeignete Acetatfasern sind 2,5- oder Triacetatfasern je nach dem gewünschten Hydrophiliegrad. Bevorzugte Synthesefasern bestehen aus Polyethylenterephthalat, Polyamid-6, Polyamid-6,6 oder modifiziertem Polyacrylnitril.

Wegen der hohen Stabilität gegen Umgebungseinflüsse und der Möglichkeit, bei der Herstellung mechanische und chemische Eigenschaften bedarfsgerecht zu modifizieren, sind synthetische, insbesondere thermoplastische, Fasern gegenüber den natürlichen bevorzugt.

Die durch die Auflagen ausgeübten Funktionen, insbesondere die Spreitfunktion, setzen voraus, daß die Fasermaterialien der Auflagen von den zu spreitenden Flüssigkeiten benetzt werden.

Diese Benetzbarkeit ist bei der Verwendung vollsynthetischer Fasern, insbesondere solchen aus unpolaren Monomeren, in der Regel von Natur aus nicht gegeben, wenn die flüssige Phase, d.h. die zu prüfende Analytlösung, im wesentlichen wässrig ist. In diesen Fällen ist es zweckmäßig, das Fasermaterial zu hydrophilieren.

Zur Hydrophilierung können sie beispielsweise mit einem oberflächenaktiven Mittel behandelt werden, sodaß sich auf den Fasern des Textilmaterials ein wirksamer Überzug des oberflächenaktiven Mittels abscheidet.

Bevorzugt zur Hydrophilierung von erfindungsgemäßen Auflagen aus hydrophoben Fasern sind N-Acyl-N-alkylglycinate der Formel I

$$R\text{-CO-N}(R^1)\text{-CH}_2\text{-COOMe} \tag{I}$$

worin R einen aliphatischen Rest mit 9 bis 23 C-Atomen, insbesondere mit 11 bis 17 C-Atomen bedeutet, der gesättigt ist oder eine bis drei Doppelbindungen aufweist

$R^1$ Wasserstoff oder Niederalkyl mit 1 bis 4 C-Atomen und

Me ein Wasserstoff- oder Metallatom ist.

Insbesondere ist R die aliphatische Kette der Laurinsäure, der Myristinsäure, der Palmitinsäure, der Stearinsäure der Palmitoleinsäure, der Oleinsäure (Ölsäure), der Linolsäure, der Linolensäure und deren Isomeren.

Sehr vorteilhaft ist auch der Einsatz eines N-Acyl-N-alkylglycinat-Gemisches in dem die Reste R bezüglich ihrer Struktur und ihres Anteils in der Mischung der Struktur und dem Anteil ihres Vorkommens den Alkylresten natürlicher Fettsäuren, z.B. der Talgfett oder der Kokosfettsäure entsprechen. Im technischen Sprachgebrauch werden derartige Alkylgemische zum Beispiel als "Talgfettalkyl" oder "Kokosfettalkyl" bezeichnet.

Für $R^1$ stehende Niederalkylgruppen sind vorzugsweise linear und haben 1 bis 4, insbesondere 1 oder 2 C-Atome. Besonders bevorzugt für $R^1$ ist die Methylgruppe.

Das Metallatom Me wird zweckmäßigerweise so gewählt, daß das Glycinat der Formel I wasserlöslich ist. Geeignet sind insbesondere die Alkalimetalle, vorzugsweise Natrium und Kalium.

Als Beispiel einer zur Hydrophilierung erfindungsgemäßer Auflagen besonders bevorzugten Verbindung der Formel I sei Natrium-oleoyl-sarcosinat genannt.

Zur Erzielung eines hydrophil wirksamen Überzugs auf erfindungsgemäßen Auflagematerialien ist in der Regel eine Menge von 0,01 bis 2 Gew.-%, vorzugsweise von 0,03 bis 0.5 Gew.-% des Glycinats, bezogen auf das Gewicht des Auflagematerials vor der Imprägnierung ausreichend.

Alternativ kann man von Natur aus hydrophobe Auflagen mit einem leicht oxidierbaren Metall oder Halbmetall, wie zum Beispiel mit Al, Si, Ti, V, Cr, Mn, Fe, Co, Ni, Zn, Ga, Ge, Zr, Nb, Cd, In, Sn oder Sb vorzugsweise mit Al, Si, Ti oder Zr, insbesondere mit Al, bedampfen und dieses anschließend zu einem hydrophilen Oxidfilm oxidieren, wie es in der Deutschen Patentanmeldung Nr. 19753848 vorgeschlagen wird.

Handelsübliche textile Flächengebilde, die, insbesondere nach einer Hydrophilierung, erfindungsgemäß eingesetzt werden können, sind beispielsweise das monofile Polyestergewebe Type PE nn HC oder das monofile Nylongewebe Type NY nn HC oder HD der Firma ZBF Mesh+Technology, Rüschlikon, CH, wobei nn von 20 bis 150 variiert, insbesondere Polyestergewebe Type PE 38 HC oder das monofile Nylongewebe Type NY 41 HC. Sehr gut geeignet für den erfindungsgemäßen Einsatz sind auch die "Viledon® - Vliesstoffe FO 2451, insbesondere der Typ FO 2451/121, der Firma Freudenberg, Weinheim, BRD.

Selbstverständlich eignen sich als erfindungsgemäße funktionelle Auflage auch alle textilen Flächengebilde mit den oben genannten Strukturmerkmalen, die aufgrund ihrer Materialeigenschaften oder anderer als den genannten Ausrüstungsbehandlungen, beispielsweise einer Corona-Plasma-Behandlung, einer Plasma Chemical Vapor Deposition oder Beschichtung mit Nanokompositen, eine ausgesprochene Spreitwirkung aufweisen.

Die erfindungsgemäßen funktionellen Auflagen werden im Rahmen der oben angegebenen Parameter so konstruiert, daß sie ohne vollflächige Befestigung in Kontakt mit den Nachweisschichten bleiben. Sie werden lediglich, wie oben beschrieben, außerhalb der Nachweisschicht, zweckmäßigerweise über einen Abstandshalter, auf dem Träger fixiert. Im Gegensatz zu der aus der DE-A-30 42 857 (Seite 10) bekannten Lehre zur Herstellung von Teststreifen werden sie nicht in die Nachweisschichten eingepreßt oder mit ihnen verklebt. Eine derartige feste Verbindung macht die beschriebene Spreitfunktion unmöglich.

Die erfindungsgemäßen funktionellen Auflagen weisen sehr günstige Spreiteigenschaften auf, d.h. sie eignen sich ausgezeichnet zur gleichmäßigen Verteilung von flüssigen Analytproben auf Nachweisschichten. Sie zeigen aufgrund ihrer zweckmäßigen Kombination von Durchlässigkeit und Flüssigkeitsleitvermögen eine deutliche Überlegenheit gegenüber bisher beschriebenen Auflagen und Abdeckungen und sind dadurch in der Lage, auch mehrere nebeneinander angeordnete Nachweisflächen von einem Auftragspunkt aus gleichmäßig mit Probenmaterial versorgen.

Die mit der erfindungsgemäßen funktionellen Auflage versehenen Teststreifen können daher vorteilhafterweise zur gleichzeitigen Bestimmung mehrerer verschiedener Analyte auf nebeneinander liegenden Nachweisfeldern eingesetzt werden, wobei die minimale Probenmenge unter 10 µl, typischerweise im Bereich von 4 bis 6 µl liegt, eine ausgezeichnete gleichmäßige Verteilung der Probe auf die verschiedenen Nachweisfelder erfolgt und ein mehrfacher Überschuß der Probe nicht schadet. Es hat sich nämlich gezeigt, daß bei der Verwendung eines Teststreifens, der unter Einsatz der oben genannten bevorzugten Materialien konstruiert wurde, ein Probenüberschuß von dem Streifen nicht aufgenommen wird sondern über der Auftragsstelle stehen bleibt. Ein weiterer erheblicher Vorteil der erfindungsgemäßen Konstruktionen ist es, daß der Teststreifen "selbstdosierend" ist. Bringt man seine Auftragsstelle mit einem auf der Fingerbeere stehenden oder an ihr hängenden Blutstropfen in Berührung, so entnimmt sich der Streifen nur die Menge, die zur Durchtränkung der Nachweisschicht(en) erforderlich ist, der Rest bleibt am Finger.

Die Erfindung betrifft auch ein Verfahren zur Herstellung einer Konstruktion aus einem flächenförmigen flexiblen Objekt und einer flächenförmigen funktionellen Auflage für zumindest einen Bereich des flächenförmigen, flexiblen Objekts, wobei die Auflage mit dem Objekt nicht vollflächig verbunden ist, und wobei die Auflage beim Biegen des Verbunds, bestehend aus flexible Objekt und Auflage, nach der Seite, auf der sich die Auflage befindet, keine Falte bildet, das dadurch gekennzeichnet ist, daß man ein flächenförmiges Auflageelement, oder mehrere flächenförmige Auflageelemente, die, sich überlappend, gemeinsam die Auflage bilden, auf dem flexiblen Objekt so befestigt, daß ein Teil ihrer Fläche gegenüber der von diesem Teil abgedeckten Objektfläche in Richtung der beim Biegen des Objekts erzeugten Krümmung frei verschiebbar ist.

Vorzugweise werden bei diesem Verfahren flächenförmige Auflageelemente eingesetzt, die aus einem der oben genannten bevorzugten Textilmaterial bestehen.

Besonders bevorzugt ist es, flächenförmige Auflageelemente einzusetzen, die mit einem N-Acyl-N-alkyl-glycinat der Formel I hydrophiliert sind.

Neben der erfindungsgemäßen funktionellen Auflage mit der beschriebenen erfindungsgemäßen Befestigung, insbesondere in Kombination mit den oben genannten Auflagematerialien und der bevorzugten Hydrophilierungsausrüstung, ist auch ein Teststreifen Gegenstand der vorliegenden Erfindung, aus einem flexiblen flächenförmigen Träger, auf dem ein oder mehrere Testfelder nebeneinander angeordnet sind, die jeweils eine oder mehrere übereinanderliegende Nachweisschichten tragen, der dadurch gekennzeichnet ist, daß die Testfelder durch eine oben beschriebene erfindungsgemäße, auf ihnen lose aufliegende funktionelle Auflage, die in der erfindungsgemäßen Weise auf dem Träger fixiert ist, abgedeckt sind.

Vorzugsweise soll der erfindunggemäße Teststreifen diagnostischen Zwecken dienen und weist mindestens zwei unmittelbar aneinandergrenzende - wobei in der Praxis auch hierbei ein mikroskopischer Spalt von ca. 5-10 µm vorhanden ist - oder durch einen Spalt getrennte ein- oder mehrschichtige Testfelder für den gleichen oder verschiedene Analyte auf.

Die auf den Testfeldern angebrachten Nachweisschichten enthalten Reagenzien für den Nachweis eines diagnostisch verwertbaren Analyts. Der Nachweis des gleichen Analyts auf zwei getrennten Testfeldern kann von Interesse sein, wenn die Nachweisschichten eine qualitative, halbquantitative oder quantitative Beurteilungen von sehr unterschiedlichen Konzentrationen des Analyts ermöglichen sollen, oder wenn einfach eine Beurteilung der Reproduzierbarkeit des Meßergebnisses erwünscht ist. Von besonderem Interesse ist natürlich der Fall, wo die beiden Nachweisschichten den gleichzeitigen qualitativen Nachweis oder eine halbquantitative oder quantitative Bestimmung von zwei verschiedenen, insbesondere diagnostisch verwertbaren, Analyten gestatten.

Besonders praktisch, insbesondere wenn der Teststreifen zwei Testfelder aufweist, ist deren Abdeckung durch eine erfindungsgemäße Auflage der oben beschriebenen, in Fig 6 veranschaulichten Art, bei der die Testfelder durch zwei Auflageelemente abgedeckt werden, die so auf dem Teststreifen fixiert sind, daß ihre verschiebbaren Bereiche gegeneinander gerichtet sind und sich überlappen. Dabei wird eine optimale Verteilung der Probe dann erreicht, wenn die Überlappung der beiden Auflageelemente über der Trennungslinie zwischen den beiden Testfeldern und vorzugsweise symmetrisch dazu liegt.

Sinnvollerweise wird in diesem Fall die Befestigung der Auflageelemente im Befestigungsbereich vollflächig ausgeführt.

Vorzugsweise sind die Testfelder auf dem Testträger in Richtung seiner Längachse hintereinander montiert und die Auflageelemente, in der gleichen Richtung gesehen, in einem vor und einem hinter den Testfeldern gelegenen Flächenbereich auf dem flexiblen Träger fixiert.

Weiterhin ist es bevorzugt, daß die Auflageelemente auf Abstandshaltern fixiert sind, die etwa die Dicke der Nachweisschichten haben.

Schließlich hat es sich als zweckmäßig erwiesen, wenn die Anordnung von Nachweisschichten und Auflagen auf dem Teststreifen mit einem inerten flächenförmigen Material so abgedeckt ist, daß nur im Bereich der Überlappung der Auflageelemente in Richtung der Längsachse des Teststreifens gesehen, eine für den Probenauftrag ausreichende Strecke freibleibt, die in der Regel 2 bis 5 mm beträgt.

Der Träger des Teststreifens besteht aus einem transparenten Material und/oder weist im Bereich der Testfelder Durchbrechungen gleicher oder unterschiedlicher Form auf, durch die die Unterseite der Nachweisschichten inspiziert werden können.

Vorteilhaft, insbesondere für eine Automatisierung der Testauswertung ist es, wenn der Träger des Teststreifens Justierungsmarkierungen in Form von zusätzlichen Bohrungen, Ausstanzungen oder Kerben aufweist.

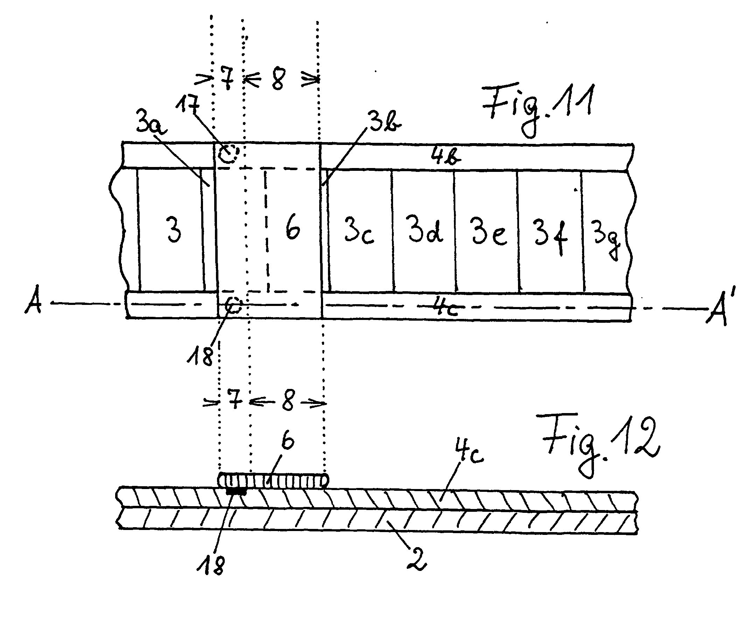

Die Figur 8 zeigt eine perspektivische Aufsicht, die Figur 9 einen Schnitt längs der Schnittlinie A-A' in Figur 8, die Figur 10 eine Aufsicht auf die Unterseite einer Ausführungsform eines erfindungsgemäßen Teststreifens mit zwei unmittelbar aneinander grenzenden Nachweisflächen und einer erfindungsgemäßen Auflage in erfindungsgemäßer Befestigung. Auch diese Darstellung erfolgt ohne Maßstab um den Aufbau klar ersichtlich machen zu können. Eine konkrete Dimensionierung dieser Ausführungsform kann dem Ausführungsbeispiel entnommen werden.

Der in Fig. 8 perspektivisch, in Fig. 9 im Schnitt und in Fig. 10 von unten gezeigte erfindungsgemäße diagnostische Testträger (1) besitzt die Form eines Teststreifens. Auf einer Tragschicht (2) befinden sich, unmittelbar aneinander grenzend zwei Nachweisschichten (3,3a), die durch die Auflageelemente (6,6a) überdeckt sind. Neben den Nachweisschichten (3,3a) sind die Auflageelemente (6,6a) mittels Abstandhaltern (4,4a) und Adhäsionsschichten (5,5a) auf der Tragschicht (2) befestigt. Diese Abstandhalter können in der Praxis auch Schmelzkleberflächen oder zweiseitig klebende Bänder sein, die die Auflagen (6,6a) auf der Tragschicht (2) fixieren. Idealerweise besitzen die Abstandhalter mit ihren Klebeflächen in etwa die gleiche Dicke wie die Nachweisschichten (3,3a). Der hier dargestellte Aufbau weist ferner Abdeckungen (10,10a) auf, die auf der Tragschicht (2) und den Auflageelementen (6,6a) befestigt sind. Sie sind so angeordnet, daß sie die über die Nachweisschichten (3,3a) hinausragenden Bereiche und einen Teil der über den Nachweisschichten liegenden Fläche der Auflagen (6,6a) überdecken. Sie lassen jedoch den über der Grenze der Nachweisfelder liegenden Überlappungsbereich der Auflageelemente (6,6a) frei. Dieser Bereich stellt die Probenauftragsstelle (11) dar. Hierauf wird die zu untersuchende Probenflüssigkeit aufgegeben. Sofern die Abdeckungen transparent sind, können auf ihnen noch über den äußeren

Begrenzungen der Nachweisfelder Markierungen (12,12a) angebracht werden, an denen der Anwender erkennen kann, ob die Nachweisfelder vollständig mit der Probenflüssigkeit gesättigt worden sind. Ist dies der Fall, so hat die Probenmenge ausgereicht, andernfalls besteht der Verdacht, daß die Probenmenge zu gering war und möglicherweise eine Fehlmessung erfolgt. Die linke Abdeckung (10) enthält aufgedruckt Pfeile (13), die dem Anwender zeigen, mit welchem Ende der Testträger (1) in ein Meßgerät gelegt oder geschoben werden soll. Das Positionierloch (14) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (14) hineinragt und so den Testträger (1) an einer vorbestimmten Stelle festhält.

Die Figur 10 zeigt die Unterseite des erfindungsgemäßen Teststreifens mit der im Träger (2) angebrachten Positionierungsbohrung (14) und den unterschiedlich geformten Beobachtungsöffnungen (15 und 16), durch die die Nachweisschichten inspiziert und vermessen werden können.

In einem erfindungsgemäßen diagnostischen Testträger kommen für die Tragschicht insbesondere solche Materialien in Frage, die die zu untersuchenden Flüssigkeiten nicht aufnehmen. Dies sind sogenannte nicht-saugfähige Materialien, wobei Kunststoffolien beispielsweise aus Polystyrol, Polyvinylchlorid, Polyester, Polycarbonat oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetat verwendet werden können. Als weitere Trägermaterialien eignen sich Metallfolien oder Glas.

Für eine Nachweisschicht ist es im Gegensatz hierzu erforderlich, solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke, Membranen oder sonstige poröse Kunststoffmaterialien oder quellfähige Materialien, wie Gelatine- oder Dispersionsfilme, die als Schichtmaterialien verwendet werden können. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf oder in einer Schicht. Es sind jedoch auch Fälle vorstellbar, für die es vorteilhafter ist, die Reagenzien auf mehrere saug- oder quellfähige Materialschichten zu verteilen, die dann übereinander, sich vollflächig berührend angeordnet sind. Der im folgenden verwendete Begriff "Nachweisschicht" soll sowohl solche Fälle umfassen, bei denen sich die Reagenzien entweder nur in oder auf einer Schicht oder in zwei oder noch mehr, wie vorstehend beschrieben, angeordneten Schichten befinden.

Bevorzugte Materialien für die Nachweisschicht sind Papiere oder poröse Kunststoffmaterialien, wie Membranen. Hiervon besonders bevorzugt sind asymmetrisch poröse Membranen, die vorteilhafterweise so angeordnet sind, daß die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen ganz besonders bevorzugt. Hervorragend geeignet sind insbesondere Polyamid 66-Membranen und hydrophilisierte asymmetrische Polysulfonmembranen. Die Reagenzien zur Bestimmung des nachzuweisenden Analyts sind in der Regel durch Imprägnierung in die vorstehend genannten Materialien eingebracht oder durch Beschichtung einseitig aufgebracht worden. Bei Beschichtung asymmetrischer Membranen wird vorteilhafterweise die feinporige Seite beschichtet.

Für die Nachweisschicht kommen jedoch auch sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich. Welche Reagenzien zur Bestimmung eines bestimmten Analyts eingesetzt werden können sind dem Fachmann bekannt. Dies muß hier nicht näher ausgeführt werden.

Ein weiteres Beispiel für eine erfindungsgemäß bevorzugte Nachweisschicht ist eine Filmschicht, wie sie in WO-A-92 15 879 beschrieben ist. Diese Schicht wird aus einer Dispersion oder Emulsion eines polymeren Filbildners hergestellt, welche zusätzlich in homogener Verteilung ein Pigment, ein Quellmittel und das Nachweisreagenz enthält. Als polymere Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z.B. von Butadien, Styrol oder Maleinsäureester geeignet. Titandioxid ist ein für den Film besonders geeignetes Pigment. Das verwendete Quellmittel soll besonders gute Quelleigenschaften aufweisen, wobei Methylvinylethermaleinsäure-Copolymer besonders empfohlen wird. Welche Reagenzien zur Bestimmung eines bestimmten Analyts eingesetzt werden, bleibt dem Fachmann überlassen.

Ganz besonders bevorzugt wird in einem erfindungsgemäßen diagnostischen Testträger ein Testfeld als Nachweisschicht eingesetzt, das aus zwei Schichten aufgebaut ist. Dieses Testfeld umfaßt eine transparente Folie, auf die in dieser Reihenfolge eine erste und eine zweite Filmschicht übereinanderliegend aufgebracht sind. Wesentlich ist, daß die auf der transparenten Folie befindliche erste Schicht im feuchten Zustand bedeutend weniger lichtstreuend ist als die darüberliegende zweite Schicht. Die nicht beschichtete Seite der transparenten Folie wird als Nachweisseite bezeichnet und die Seite der zweiten Schicht, die der Seite gegenüberliegt, mit der die zweite Schicht auf der ersten aufliegt, wird als Probenaufgabenseite bezeichnet.

Die Filmschichten werden aus Dispersionen oder Emulsionen polymerer Filmbildner hergestellt. Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z. B. von Butadien, Styrol oder Maleinsäureester geeignet.

In dem Testfeld befinden sich die zwei genannten Filmschichten auf einer transparenten Folie. Hierfür kommen insbesondere solche Kunststofffolien in Betracht, die flüssigkeitsundurchlässig sind. Polycarbonatfolie hat sich als besonders bevorzugt erwiesen.

Die beiden Filmschichten können aus Beschichtungsmassen hergestellt werden, die den gleichen polymeren Filmbildner enthalten oder sie können aus Beschichtungsmassen erzeugt werden, die unterschiedliche polymere Filmbildner enthalten.

Sofern besondere Testaufgaben und/oder Testbedingungen vorliegen, wie z.B. bei der Bestimmung von Glukose in Vollblut, ist es zweckmäßig, die Schichten so auszubilden, daß sie außer guter Erythrocytenseparation auch optische Merkmale aufweisen, die die Beobachtung der Nachweisreaktion erleichtern und die Exaktheit der Beurteilung und der meßtechnischen Erfassung verbessern.

Hierzu enthält die erste Schicht zweckmäßigerweise ein Quellmittel und gegebenenfalls einen schwach lichtstreuenden Füllstoff, die zweite Schicht ein Quellmittel und wenigstens ein stark lichtstreuendes Pigment. Daneben kann die zweite Schicht auch nicht-poröse Füllstoffe sowie poröse Füllstoffe, wie Kieselgur, in geringen Mengen enthalten, ohne dadurch für Erythrozyten durchlässig zu werden.

Durch Zugabe eines gut quellenden Quellmittels (das heißt, einer Substanz, die unter Aufnahme von Wasser ihr Volumen vergrößert) erhält man nicht nur Schichten, die relativ schnell von Probenflüssigkeit penetriert werden, sondern die trotz dieser Öffnungswirkung des Quellmittels gute Erythrozyten- und außerdem auch Blutfarbstoffabtrenneigenschaften besitzen. Die Quelleigenschaften sollten so gut sein, daß für einen Test, bei dem die Geschwindigkeit der Farbbildung - wie beispielsweise einer Glucosenachweisreaktion - überwiegend von der Penetration der Probenflüssigkeit durch die Schicht abhängt, die optisch nachweisbare Reaktion nach maximal einer Minute meßbar ist. Als besonders geeignete Quellmittel haben sich Xanthangum und Methylvinylethermaleinsäure-Copolymer erwiesen.

Kieselgur wird auch als Diatomeenerde bezeichnet. Es handelt sich um aus den Kieselsäuregerüsten der Diatomeenarten entstandene Ablagerungen, die an verschiedenen Orten abgebaut werden. Die bevorzugt eingesetzte Kieselgur hat einen mittleren Teilchendurchmesser von 5-15 µm, wobei diese Werte mit einem Laser-Granulometer Typ 715 bestimmt wurden, welches von der Firma Pabisch, München, Bundesrepublik Deutschland, vertrieben wird.

Der stark lichtstreuende Pigmentanteil der zweiten Schicht liegt bei mindestens 25 Gewichts-%, bezogen auf die getrocknete und einsatzbereite Doppelschicht des Testfeldes. Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente.

Die erste Filmschicht soll entweder keine oder solche Füllstoffe enthalten, deren Brechungsindex nahe beim Brechungsindex von Wasser liegt. Als besonders geeignet hierfür haben sich gefällte Kieselsäuren, Siliziumdioxid, Silikate und Aluminiumsilikate erwiesen. Ein Natriumaluminiumsilikat mit dem Handelsnamen Transpafill® ist besonders bevorzugt.

Die zweite Schicht soll möglichst stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 µm haben sich als besonders vorteilhaft erwiesen. Leicht verarbeitbare Titandioxid-Typen in der Anatas-Modifikation sind ganz besonders bevorzugt.

Reagenzsysteme zum Nachweis bestimmter Analyte durch Farbbildung sind dem Fachmann bekannt. Es ist möglich, daß sich sämtliche Komponenten des Reagenzsystems in einer Filmschicht befinden. Es ist aber auch möglich, daß die Komponenten des Reagenzsystems auf beide Filmschichten verteilt sind. Vorteilhafterweise befindet sich das farbbildende Reagenzsystem wenigstens zum Teil in der ersten Filmschicht.

Unter Farbbildung wird im Rahmen der vorliegenden Erfindung nicht nur der Übergang von weiß nach farbig verstanden, sondern auch jede Farbveränderung, wobei natürlich solche Farbveränderungen besonders bevorzugt sind, die mit einer möglichst großen Verschiebung der maximalen Absorptionswellenlinie ($\lambda$ max) einhergehen.

Für die Optimierung des Testfeldes in dem erfindungsgemäßen diagnostischen Testträger hat es sich als besonders vorteilhaft erwiesen, wenn beide Filmschichten ein nicht hämolysierendes Netzmittel enthalten. Neutrale, das heißt, nicht geladene Netzmittel sind hierfür besonders geeignet. Ganz besonders bevorzugt wird N-Octanoyl-N-methyl-glucamid. Zusätzlich können in den Filmschichten weitere Netzmittel, die die Homogenität der Beschichtungen fördern, wie beispielsweise Natrium-N-methyl-N-oleoyl-taurat enthalten sein.

Zur Herstellung eines Testfeldes eines erfindungsgemäßen diagnostischen Testträgers werden die jeweiligen Filmschichten jeweils nacheinander aus einer homogenen Dispersion der genannten Bestandteile hergestellt. Hierzu verwendet man als Unterlage für das Ausformen der Beschichtungsmasse für die erste Filmschicht die transparente Folie. Nach Aufbringen der Beschichtungsmasse für die erste Filmschicht in einer bestimmten Schichtdicke wird die Schicht getrocknet. Danach wird auf diese Schicht die Beschichtungsmasse für die zweite Schicht ebenfalls in einer dünnen Schichtdicke aufgebracht und getrocknet. Nach dem Trocknen sollte die Dicke der ersten und zweiten Filmschicht zusammen maximal 0,2 mm, bevorzugt maximal 0,12 mm, besonders bevorzugt maximal 0,08 mm betragen.

Die Befestigung kann nach dem Fachmann aus der Testträgertechnologie bekannten Methoden erfolgen. Beispielsweise kann die Befestigung mittels Schmelzkleber oder härtendem Kaltkleber erfolgen. Dabei ist eine punktuelle oder gerasterte Verklebung vorteilhaft, da der kapillaraktive Flüssigkeitstransport in diesem Fall besonders gut möglich ist. Als vorteilhaft haben sich auch doppelseitig klebende Streifen erwiesen. In allen Fällen ist es jedoch wichtig, daß die Befestigung der Auflage auf der Tragschicht so erfolgt, daß von der Nachweisschicht ein kapillaraktiver Flüssigkeitstransport in den Teil der Auflage möglich ist, der auf der Tragschicht befestigt ist Dieser kapillaraktive Flüssigkeitstransport muß insbesondere dann möglich sein, wenn die Nachweisschicht mit Flüssigkeit gesättigt ist. Für die Verarbeitung ganz besonders geeignet haben sich Klebebänder mit Natur- oder Synthesekautschuk erwiesen. Ganz besonders vorteilhaft ist es, wenn das Mittel, das zur Befestigung der Auflage auf der Tragschicht dient, etwa die gleiche Dicke wie die Nachweisschicht(en) hat. Es dient dann quasi als Abstandshalter, um die erfindungsgemäße Auflage auch außerhalb des Bereiches der Nachweisschicht(en) insgesamt auf einer durchgehenden Fläche eben zu halten.

Zur Bestimmung des in der Probenflüssigkeit nachzuweisenden Analyts ist in dem erfindungsgemäßen diagnostischen Testträger die Nachweisschicht, zumindest aber sind die Reaktionsbezirke, das heißt, Reagenz tragende Bereiche der Nachweisschicht(en), die auf Signalbildung hin beobachtet und vermessen werden können, durch die Tragschicht sichtbar. Dies kann, wie oben bereits ausgeführt, dadurch erreicht werden, daß die Tragschicht transparent ist. Es ist aber auch möglich, daß die Tragschicht eine Lochung aufweist, die von der Nachweisschicht oder den Nachweisschichten überdeckt ist. Durch die Lochung ist dann die Nachweisschicht oder sind die Nachweisschichten zumindest aber die Reaktionsbezirke der Nachweisschichten sichtbar. In einer bevorzugten Ausführungsform des erfindungsgemäßen diagnostischen Testträgers befindet sich in der Tragschicht unterhalb einer Nachweisschicht ein Loch, durch das die Nachweisschicht oder ein Reaktionsbezirk beobachtbar ist. Das Loch besitzt einen etwas kleineren Durchmesser als die kleinste Längenausdehnung der Nachweisschicht, so daß die Nachweisschicht außerhalb des Loches auf der Tragschicht aufliegt und dort befestigt sein kann. Vorteilhafterweise ist die Nachweisschicht durch beidseitig daneben angeordnete doppelseitige Klebebänder und die über der Nachweisschicht liegende erfindungsgemäße Auflage und deren Befestigung auf der Tragschicht ausreichend fixiert. Bevorzugt ist jedoch die Nachweisschicht selbst auch mittels dünnem Klebeband auf der Tragschicht befestigt.

Durch ein Loch können aber auch mehrere Reaktionsbezirke einer Nachweisschicht sichtbar sein.

Die Lochung eines erfindungsgemäßen diagnostischen Testträgers kann auch aus zwei oder mehr Löchern bestehen, die zur Bestimmung von Analyt (einem oder mehreren Analyten) genutzt werden können. Über den Löchern können verschiedene Nachweisschichten angeordnet sein oder auch nur eine Nachweisschicht mit mehreren Reaktionsbezirken, so daß durch je 1 Loch eine Nachweisschicht oder je ein Reaktionsbezirk beobachtet werden kann. Es ist auch möglich, daß durch ein Loch mehrere Reaktionsbezirke beobachtet werden können.

Über der erfindungsgemäßen Auflage des erfindungsgemäßen diagnostischen Testträgers kann zweckmäßigerweise eine inerte Abdeckung aus probenundurchlässigem, in der Regel wasserundurchlässigem und nicht saugfähigem Material, so angeordnet werden, daß der Bereich der Auflage außerhalb der Nachweisschicht abgedeckt ist. Idealerweise ragt die Abdeckung auch noch in den Bereich der Nachweisschicht hinein, läßt jedoch auf jeden Fall einen mittleren Teil der erfindungsgemäßen Auflage, die die Nachweisschicht bedeckt, frei. Dieser freie Teil der Auflage wird als Probenauftragsstelle bezeichnet.

Als Abdeckung haben sich Kunststofffolien als besonders vorteilhaft erwiesen. Wenn Abdeckung und die erfindungsgemäße Auflage unterschiedliche Farben haben, beispielsweise weiß und gelb oder weiß und rot, kann damit die Stelle sehr gut kenntlich gemacht werden, auf die zu untersuchende Probenflüssigkeit aufgegeben werden soll.

Auf der Abdeckung kann auch beispielsweise mit einem oder mehreren aufgedruckten Pfeilen verdeutlicht werden, in welcher Richtung, das heißt, mit welchem Ende ein erfindungsgemäßer diagnostischer Testträger in ein Meßgerät gelegt oder geschoben werden soll.

Eine Probenauftragsstelle kann besonders einfach durch eine Abdeckung mittels zweier bandförmiger Kunststofffolien erreicht werden, die einen bandartigen Bereich der die Nachweisschicht überdeckenden erfindungsgemäßen Auflage frei lassen. Wenn 2 oder mehr Probenauftragsstellen vorgesehen werden, sind 3 oder mehr bandförmige Kunststofffolien zu verwenden. Die zur Abdeckung verwendeten Folien sind auf der erfindungsgemäßen Auflage und gegebenenfalls auf der Tragschicht befestigt. Für eine solche Befestigung eignen sich Schmelzkleber, die vorzugsweise punktuell oder gerastert auf der Tragschicht oder der Unterseite der Abdeckung aufgebracht sind oder Klebebänder, wenn die Folien nicht selbst klebefähig sind. Die Probenauftragsstelle befindet sich vorzugsweise über der Lochung in der Tragschicht, durch die eine Signalbildung in der Nachweisschicht beobachtet werden kann.

Die vorliegende Erfindung betrifft auch ein Verfahren zur Herstellung eines diagnostischen Teststreifens bei dem man auf einem gegebenenfalls transparenten oder mit Inspektionsöffnungen versehenen Träger ein- oder mehrschichtige Nachweisfelder aufbringt, diese mit einer seitlich von den Testfeldern fixierten auf den Testfeldern lose aufliegenden funktionellen Auflage überdeckt und gegebenenfalls noch eine inerte Abdeckung, die nur die Probenauftragsstelle freiläßt aufbringt, das dadurch gekennzeichnet ist, daß man ein flächenförmiges Auflageelement, oder mehrere flächenförmige Auflageelemente, die, sich überlappend, gemeinsam die Auflage bilden, auf dem Teststreifen neben dem (den) Testfeld(ern) so befestigt, daß ein Teil ihrer Fläche das (die) Testfeld(er) überdeckt und gegenüber dem (den) Testfeld(ern) in Richtung der beim Biegen des Objekts erzeugten Krümmung frei verschiebbar ist.

Vorzugsweise werden bei diesem Verfahren flächenförmige Auflageelemente eingesetzt, die aus einem der oben genannten bevorzugten Textilmaterialien bestehen.

Besonders bevorzugt ist es, flächenförmige Auflageelemente einzusetzen, die mit einem N-Acyl-N-alkyl-glycinat der Formel I hydrophiliert sind.

Zur Durchführung eines Verfahrens zur Bestimmung von Analyt in flüssiger Probe mit Hilfe eines erfindungsgemäßen diagnostischen Testträgers wird Probenflüssigkeit auf die der Nachweisschicht abgewandten Seite der Auflage aufgegeben, idealerweise so viel, daß die durch die erfindungsgemäße Auflage gelangende Flüssigkeit die Nachweisschicht vollständig sättigt. Als Probenflüssigkeit kommen insbesondere Körperflüssigkeiten wie Blut, Plasma, Serum, Urin, Speichel etc. in Frage. Blut oder von Blut abgeleitete Flüssigkeiten wie Plasma oder Serum sowie Urin sind besonders bevorzugte Probenflüssigkeiten. In der Nachweisschicht kann dann bei Anwesenheit des zu bestimmenden Analyts ein Signal nachgewiesen werden. Vorteilhafterweise handelt es sich bei einem solchen Signal um eine Farbänderung, worunter sowohl Farbbildung, Farbverlust als auch Farbumschlag verstanden wird. Die Intensität der Farbänderung ist ein Maß für die Menge an Analyt in der untersuchten flüssigen Probe. Sie kann visuell oder mit Hilfe eines Gerätes, meistens reflektionsphotometrisch quantitativ ausgewertet werden, wobei in Vorversuchen geschaffene Eichkurven benutzt werden können. Alternativ kann auch eine direkte Anzeige des Analytgehalts über die Geräte-Software bewerkstelligt werden.

Ein großer Vorteil des erfindungsgemäßen diagnostischen Testträgers besteht darin, daß kein vorbestimmtes Volumen einer Probenflüssigkeit auf den Testträger aufgegeben werden muß. Überschüssige Flüssigkeit wird, wie bereits vorstehend erwähnt, den Nachweisfeldern nicht zugeleitet sondern bleibt über dem Auftragsspalt stehen. Dies ist insbesondere bei der Untersuchung von Blut oder von Blut abgeleiteten Proben, wie Plasma oder Serum, ein sehr wichtiger Aspekt.

Auch bei einer direkten Zufuhr von Blut aus der Fingerbeere erweist sich der erfindungsgemäße Teststreifen als selbstdosierend. Er entnimmt dem Blutstropfen auf der Fingerbeere ca. 5 µl, der Rest bleibt am Finger.

Durch die Abdeckung von Teilen der erfindungsgemäßen Auflage und damit der Markierung der Probenauftragsstelle wird dafür Sorge getragen, daß Flüssigkeit nur an der dafür optimalen Stelle auf die Nachweisschicht gelangen kann. In Kombination mit einer Nachweisschicht, die nur wenig Flüssigkeit aufnimmt und dennoch eine intensive Signalbildung gewährleistet, wird sichergestellt, daß bereits bei sehr kleinen Probenvolumina zuverlässige Analytbestimmungen möglich sind. Dadurch, daß der erfindungsgemäße Testträger aus nur wenigen Komponenten besteht, die einfach und schnell zusammenfügbar sind, ist er sehr preiswert herzustellen.

Bei den oben beschriebenen Ausführungsformen der vorliegenden Erfindung waren die Auflageelemente in ihrem Befestigungsbereich vollflächig, vorzugsweise über Abstandshalter, auf dem Träger fixiert. Wie oben aber bereits angegeben, ist die vollflächige Fixierung keineswegs erforderlich. Vielmehr genügt im Prinzip ein einziger, vorzugsweise genügen zwei Befestigungspunkte, die im Befestigungsbereich des Auflageelements liegen. Bei einer derartigen Ausführungsform können die Befestigungspunkte des Auflageelements im Befestigungsbereich an je einer sich in Krümmungsrichtung erstreckenden Kante des Auflageelements liegen. Der zwischen den Kanten liegende Teil des Befestigungsbereichs weist dagegen keine Befestigungspunkte auf.

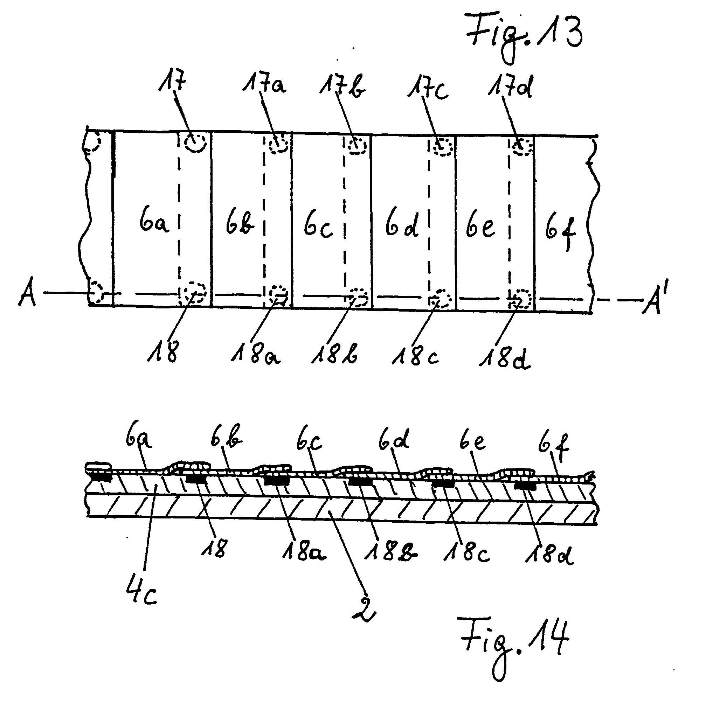

Die Figur 11 zeigt eine Aufsicht auf einen Abschnitt eines Teststreifens, Figur 12 eine Schnittdarstellung längs der Schnittlinie A-A' dieses Objekts. Der Streifen weist eine ununterbrochene Folge von Nachweisfeldern (3 bis 3g) auf, die sich in Richtung der Längachse des Teststreifens erstreckt. Beiderseits der Nachweisfelder erstrecken sich ebenfalls in Längsrichtung des Teststreifens Abstandshalterleisten (4b und 4c), auf denen das einen Teilbereich der Nachweisschicht überdeckende erfindungsgemäße Auflageelement (6) in den Punkten 17 und 18 seines Befestigungsbereichs (7) fixiert ist, im verschiebbaren Bereich (8) aber lose aufliegt.

Diese Befestigungsart wird zweckmäßigerweise dann gewählt, wenn eine langgestreckte abzudeckende Teilfläche des Objekts durch mehrere in Krümmungsrichtung hintereinander gleichsinnig oder gegensinnig angeordnete, schindelartig überlappende Auflageelemente abgedeckt werden muß: Die Figuren 13 und 14 veranschaulichen diese Ausführungsform der Erfindung.

Die Figur 13 zeigt eine Aufsicht auf einen Abschnitt eines Teststreifens, Figur 14 eine Schnittdarstellung längs der Schnittlinie A-A' dieses Objekts. Der Streifen weist, wie der in Figur 11 dargestellte, eine ununterbrochene Folge von Nachweisfeldern auf - die aus Gründen der Übersichtlichkeit in Figur 13 nicht dargestellt sind - die sich in Richtung der Längachse des Teststreifens erstreckt. Beiderseits der Nachweisfelder erstrecken sich ebenfalls in Längsrichtung des Teststreifens Abstandshalterleisten (4b und 4c), auf denen eine Reihe die Nachweisschicht überdeckender, sich schindelartig überlappender erfindungsgemäßer Auflageelemente (6a bis 6f) in den Punkten (17 bis 17d) und (18 bis 18d) fixiert ist.

Eine solche Ausführungsform von Teststreifen und Testblättern kann beispielsweise für eine Feinbestimmung des pH-Wertes mittels einer größeren Reihe verschiedener Indikatorfelder eingesetzt werden, wobei der Probenauftrag in Form einer in der Längachse des Streifens verlaufenden Auftragslinie erfolgt.

Diese Ausführungsform zeigt, daß das Prinzip der einseitigen Befestigung der Auflageelemente einer mannigfachen Variation zugänglich ist. Auch diese Varianten sind Bestandteil dieser Erfindung.

Die folgenden Ausführungsbeispiele veranschaulichen die Herstellung eines erfindungsgemäßen Testreifens und seine Verwendung.

**Beispiel 1.**

Herstellung eines erfindungsgemäßen diagnostischen Testträgers mit zwei Nachweisfeldern für die Bestimmung von Glucose in niedriger und in hoher Konzentration.

Die Herstellung eines Testträgers gemäß den Figuren 8, 9 und 10 erfolgt mit folgenden Arbeitsschritten:

Auf eine bandförmige, 50 mm breite Titandioxid-haltige Polyester-Tragschicht wird parallel im Abstand von 18,6 mm (gemessen zur linken Kante des Klebebandes) zu seiner linken Kante ein 10 mm breites doppelseitiges

EP 0 995 993 B1

Klebeband (Polyesterträger und Synthesekautschuk-Kleber) aufgebracht. Aus diesem Verbund werden mit einem Abstand von 6 mm jeweils drei Löcher, ein Positionierungsloch und zwei Inspektions- und Meßlöcher, ausgestanzt, deren Mittelpunkte auf einer senkrecht zur Längachse der Trägerstreifens liegenden Geraden liegen. Das erste Loch, das Positionierungsloch, ist kreisrund, hat einen Durchmesser von 2,6 mm und sein Mittelpunkt hat von der linken Kante des Trägerstreifens einen Abstand von 4 mm. Das zweite Loch ist ebenfalls rund mit einem Durchmesser von 4 mm, das dritte Loch ist rechteckig mit einer Kantenlänge von 3 mm in Längsrichtung des Streifens und 4 mm in Querrichtung. Das zweite und dritte Loch liegen beide in einem Mittelpunktsabstand von 5,1 mm auf dem Klebeband. der Mittelpunksabstand des zweiten Loches von der linken Kante des Trägerstreifens beträgt 21 mm.

Danach wird das Schutzpapier des doppelseitigen Klebebands abgezogen.

Zur Herstellung der ersten Nachweisschicht, die aus 2 Filmschichten aufgebaut ist, wird so vorgegangen:

A. In einem Becherglas werden die folgenden Komponenten als Reinsubstanzen oder in Form von Stammlösungen in folgender Zusammensetzung zusammengegeben und durch Rühren gemischt:

Wasser: 820,0 g

Citronensäure-1-hydrat: 2,5 g

Calciumchlorid-2-hydrat: 0,5 g

Natriumhydroxid: 1,4 g

Xanthan gum: 3,4 g

Tetraethylammoniumchlorid: 2,0 g

N-Octanoyl-N-methyl-glucamid: 2,1 g

Natrium-N-methyl-N-oleoyl-taurat 0,29 g

Polyvinylpyrrolidon (MG 25000): 3,5 g

Transpafill((Natrium-Aluminiumsilikat): 62,1 g

Polyvinylpropionat-Dispersion (50 Gew.-% in Wasser): 60,8 g

Bis-(2-hydroxyethyl)-(4-hydroximinocyclohexa-2,5-dienylidin)-ammoniumchlorid: 1,2 g

2,18-Phosphormolybdänsäure-hexanatriumsalz: 16,1 g

Pyrrolochinolin-chinon: 32 mg

Glucosedehydrogenase rec. aus Acinetobacter calcoaceticus, 1,7 MU EC 1.1.99.17: (2,4 g)

1-Hexanol: 1,6 g

1-Methoxy-2-propanol: 20,4 g

Die Gesamtmasse wird mit NaOH auf einen pH von ca. 6 eingestellt und dann mit einem Flächengewicht von 89 g/qm auf eine 125 µ dicke Polycarbonatfolie aufgetragen und getrocknet.

B. In einem Becherglas werden die folgenden Komponenten als Reinsubstanzen oder in Form von Stammlösungen in folgender Zusammensetzung zusammengegeben und durch Rühren gemischt:

Wasser: 579,7 g

Natriumhydroxid: 3,4 g

Gantrez((Methylvinylether-maleinsäure-Copolymer): 13,8 g

N-Octanoyl-N-methyl-glucamid: 3,6 g

Natrium-N-methyl-N-oleoyl-taurat 0,25 g Tetraethylammoni umchlorid: 9,7 g

Polyvinylpyrrolidon (MG 25000): 20,2 g

Titandioxid: 177,1 g

Kieselgur: 55,3 g

Polyvinylpropionat-Dispersion (50 Gew.-% in Wasser): 70,6 g

2,18-Phosphormolybdänsäure-hexanatriumsalz: 44,3 g

Kaliumhexacyanoferrat (III): 0,3 g

1-Hexanol: 1,6 g

1-Methoxy-2-propanol: 20,4 g

Die Gesamtmasse wird mit NaOH auf einen pH von ca. 6 eingestellt und dann mit einem Flächengewicht von 104 g/qm auf die wie vorstehend unter A. beschriebene beschichtete Polycarbonatfolie aufgetragen und getrocknet. Die Schichtdicke beträgt nach dem Trocknen 60 µm.

Zur Herstellung der zweiten Nachweisschicht, die ebenfalls aus 2 Filmschichten aufgebaut ist, verfährt man wie folgt:

A. In einem Becherglas werden die folgenden Komponenten als Reinsubstanzen oder in Form von Stammlösungen in den angegebenen Mengen unter Rühren gemischt:

Wasser: 820,0 g

Citronensäure-1-hydrat: 2,5 g

Calciumchlorid-2-hydrat: 0,5 g

Natriumhydroxid: 1,4 g

Xanthan gum: 3,4 g

Tetraethylammoniumchlorid: 4,22 g

N-Octanoyl-N-methyl-glucamid: 2,1 g

Natrium-N-methyl-N-oleoyl-taurat 0,29 g

Polyvinylpyrrolidon (MG 25000): 3,5 g

Transpafill((Natrium-Aluminiumsilikat): 62,1 g

Polyvinylpropionat-Dispersion (50 Gew.-% in Wasser): 60,8 g

N-(4-Nitrosophenyl)-N'-carboxymethyl-piperazin: 1,0 g

2,18-Phosphormolybdänsäure-hexanatriumsalz: 20,9 g

Pyrrolochinolin-chinon: 32 mg

Glucosedehydrogenase rec. aus Acinetobacter calcoaceticus, 1,7 MU (EC 1.1.99.17): (2,4 g)

1-Hexanol: 1,6 g

1-Methoxy-2-propanol: 20,4 g

Die Gesamtmasse wird mit Natronlauge auf einen pH-Wert von ca. 6,0 eingestellt und dann mit einem Flächengewicht von 89 g/m$^2$ auf eine 125 µm dicke Polycarbonatfolie aufgetragen und getrocknet.

B. In einem Becherglas werden die folgenden Komponenten als Reinsubstanzen oder in Form von Stammlösungen in den angegebenen Mengen unter Rühren gemischt:

Wasser: 579,7 g

Natriumhydroxid: 3,4 g

Gantrez((Methylvinylether-maleinsäure-Copolymer): 13,8 g

Tetraethylammoniumchlorid: 6,71 g

N-Octanoyl-N-methyl-glucamid: 2,74 g

Natrium-N-methyl-N-oleoyl-taurat 0,25 g

Polyvinylpyrrolidon (MG 25000): 15,6 g

Titandioxid: 136,7 g

Polyvinylpropionat-Dispersion (50 Gew.-% in Wasser): 54,6 g

N-(4-Nitrosophenyl)-N'-carboxymethyl-piperazin: 1,51 g

2,18-Phosphormolybdänsäure-hexanatriumsalz: 33,13 g

Kaliumhexacyanoferrat (III): 0,28 g

1-Hexanol: 1,6 g

1-Methoxy-2-propanol: 20,4 g

Die Gesamtmasse wird mit Natronlauge auf einen pH-Wert von ca. 6,0 eingestellt und dann mit einem Flächengewicht von 102 g/m$^2$ auf die wie vorstehend unter A beschriebene beschichtete Polycarbonatfolie aufgetragen und getrocknet. Die Schichtdicke beträgt nach dem Trocknen 55 µm.

Je ein 5 mm breiter Streifen der so hergestellten Nachweisschichten wird mit der Folienseite auf das gestanzte doppelseitige Klebeband auf der Tragschicht passgenau so aufgeklebt, das die Streifen unmittelbar aneinander grenzend nebeneinander herlaufen.

Direkt an die Nachweisschichten angrenzend werden auf beiden Seiten doppelseitige Klebebänder in der Dicke der Nachweisstreifen als Abstandshalter auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Dann wird ein 10 mm breiter Streifen eines mit Netzmittel imprägnierten monofilen Polyestergewebes (Typ PE 38 HC der Fa. ZBF Mesh+Technology, Rüschlikon, Schweiz) so auf den 9 mm breiten Abstandshalter aufgelegt, daß die Schnittkante des Gewebestreifens die Grenzlinie zwischen den Nachweisstreifen um 0,5 bis 0,6 mm überragt und durch Andrücken fixiert. Anschließend wird auf den 6 mm breiten Abstandshalter ein 10 mm breiter Streifen des gleichen Polyestergewebes so aufgelegt, daß er die Schnittkante des ersten Gewebestreifens um 1 bis 1,2 mm überlappt und durch Andrücken fixiert.

Danach werden auf beiden Seiten des Aufbaus zwei einseitige Klebebänder (PVC-Träger und Naturkautschuk-Kleber) als Abdeckungen so aufgeklebt, daß symmetrisch zur Grenzlinie der Nachweisstreifen ein Spalt von 2 bis 2,5 mm unbedeckt bleibt. Damit ist die Bandware fertiggestellt.

Die Bandware wird so in 6 mm breite Testträger geschnitten, daß die Meß- und Inspektionslöcher und das Positionierungsloch im Testträger mittig liegen.

**Beispiel 2**

Das Beispiel 1 wird wiederholt, wobei das für die erfindungsgemäße Auflage eingesetzte monofile Polyestergewebe Typ PE 38 HC mit Natrium-N-oleoyl-sarcosinat als Netzmittel imprägniert wird. Die Netzmittelmenge auf dem Gewebe beträgt 0,25 Gew.-%, bezogen auf das Gewicht des Gewebes vor der Imprägnierung.

**Beispiel 3**

Gemäß Beispiel 1 werden Teststreifen hergestellt, die in beiden Nachweisfeldern die gleichen Nachweisschichten aufweisen. Als Spreitschicht wird das in Beispiel 2 verwendete, mit 0,25 Gew.-% Natrium-N-oleoyl-sarcosinat imprägnierte Polyestergewebe Typ PE 38 HC eingesetzt. Die Streifen werden in ein für die gleichzeitige Ausmessung beider Testfelder eingerichtetes GLUCOTREND-Gerät eingeschoben, wobei sie zur Fixierung in der Meßposition einer leichten Biegung unterworfen werden.

Die Streifen werden mit steigenden Volumina EDTA-Venenblut mit 102 mg/dl Glucose getüpfelt. Je Volumen wurden 5 Serien zu 10 Teststreifen vermessen (n=5, N=50). Hieraus wurden für jedes Volumen 5 VK-Werte berechnet. (Der VK-Wert ist definiert als die relative Standardabweichung

$$VK = \text{Standardabweichung} / \text{Mittelwert}$$

und wird in % angegeben.)

Die folgende Tabelle zeigt die Mediane der Messergebnisse und die Mediane der VK-Werte der jeweils 5 Serien je Volumen:

| | Feld 1 | | Feld 2 | |
|---|---|---|---|---|
| Volumen | Median des Meßwertes [mg/dl] | Median des VK-Wertes [%] | Median des Meßwertes [mg/dl] | Median des VK-Wertes [%] |
| 3 µl | Fehler* | - | 63,4** | 9,3 |
| 4 µl | 100 | 2,5 | 102 | 2,7 |
| 5 µl | 102 | 2,3 | 103 | 2,6 |
| 10 µl | 101 | 2,2 | 102 | 2,7 |
| 15 µl | 103 | 2,4 | 102 | 2,7 |

Anmerkungen:

*:Eine ungleichmäßige Reaktionsfärbung wird auf dem Feld 1 durch die 2-LED-Optik des Gerätes (beschrieben in EP-A-819 943) erkannt. Das Gerät gibt daher nur eine Fehlermeldung, aber keinen Meßwert aus.

**:Feld 2 wird nur mit einer LED beleuchtet, sodaß eine ungleichmäßige Färbung des Testfeldes nicht erkannt wird. Demzufolge ist der VK-Wert stark erhöht. Das Gerät kann jedoch durch Vergleich beider Testfelder die Fehlermeldung "verschieden tiefe Reaktionsfarbe beider Felder" abgeben. Diese Funktion war bei der hier durchgeführten Messung abgeschaltet.

Die Versuche zeigen, daß ab einem Probevolumen von 4 µl beide Testfelder die gleiche Färbung, d.h. die gleiche Glucosekonzentration anzeigen. Hieraus erkennt man die ausgezeichnete Spreitwirkung der erfindungsgemäßen Auflage über beide Meßfelder. Bei höheren Probevolumina ändert sich der Wert nicht, da das überschüssige Probenmaterial über dem Auftragsspalt stehen bleibt. Bei kleineren Volumina ist die Benetzung und damit auch die Färbung der beiden Reaktionsfelder unvollständig, was durch die 2-LED-Optik des Feldes 1 detektiert werden kann. Durch geeignete Softwaremaßnahmen kann verhindert werden, daß solche Messungen zu einer Anzeige von (falschnegativen) Werten führen. Stattdessen wird eine Fehlermeldung angezeigt, die den Benutzer auf das zu niedrige Probenvolumen hinweist.

**Patentansprüche**

1.  Konstruktion umfassend ein flächenförmiges, flexibles Objekt und eine flächenförmige funktionelle Auflage für zumindest einen Bereich des flächenförmigen, flexiblen Objekts, die mit dem Objekt nicht vollflächig verbunden ist, und die beim Biegen des Verbunds, bestehend aus flexiblem Objekt und Auflage, nach der Seite, auf der sich die Auflage befindet, keine Falte bildet, **dadurch gekennzeichnet, daß** die Auflage aus einem oder mehreren flächenförmigen Auflageelementen besteht, die auf dem flexiblen Objekt so befestigt sind, daß ein Teil ihrer Fläche gegenüber der von diesem Teil abgedeckten Objektfläche in Richtung der beim Biegen des Objekts erzeugten Krümmung überlappend angeordnet ist, damit die Fläche frei verschiebbar ist.

2.  Konstruktion gemäß Anspruch 1, **dadurch gekennzeichnet, daß** die Befestigung des Auflageelements auf dem flexiblen Objekt durch mindestens einen, vorzugsweise mindestens zwei, Befestigungspunkte erfolgt, die in einem zusammenhängendem Flächenbereich (Befestigungsbereich) des Auflageelements liegen, der sich zwischen den in Krümmungsrichtung liegenden Kanten des Auflageelements erstreckt und dessen Abgrenzung von dem freibeweglichen Teil oder den frei verschiebbaren Teilen des Auflageelements im wesentlichen geradlinig und quer zur Krümmungsrichtung verläuft.

3.  Konstruktion gemäß mindestens einem der Ansprüche 1 und 2, **dadurch gekennzeichnet, daß** der Befestigungsbereich ein Ende des Auflageelements umfaßt.

4.  Konstruktion gemäß mindestens einem der Ansprüche 1 bis 3, **dadurch gekennzeichnet, daß** die Auflage an ihrem verschiebbaren Ende mit einer Auflage gleicher oder beliebig anderer Befestigungsart überlappt.

5.  Konstruktion gemäß mindestens einem der Ansprüche 1 bis 4, **dadurch gekennzeichnet, daß** die Auflage aus zwei Auflageelementen besteht, deren verschiebbare Bereiche gegeneinander gerichtet sind und sich überlappen.

6.  Konstruktion gemäß mindestens einem der Ansprüche 1 bis 5, **dadurch gekennzeichnet, daß** die Auflageelemente auf einer Länge von 0,5 bis 2,5 mm überlappen.

7.  Konstruktion gemäß mindestens einem der Ansprüche 1 bis 6, **dadurch gekennzeichnet, daß** die Auflage aus einem offenporigen Flächengebilde aus einem Textilmaterial besteht, das nicht kapillaraktiv ist und daß die Konstruktion und das Material des offenporigen Flächengebildes und/oder seine Ausrüstung so gewählt wird, daß es auf einer Unterlage aufliegend, 10 μl Wasser auf eine Fläche von mehr als 300 mm$^2$ spreitet.

8.  Konstruktion gemäß mindestens einem der Ansprüche 1 bis 7, **dadurch gekennzeichnet, daß** das Textilmaterial eine Dicke von 20 bis 200, insbesondere 30 bis 100 μm und/oder einem Porenvolumen von 30 bis 85, insbesondere 40 bis 75 % aufweist.

9.  Konstruktion gemäß mindestens einem der Ansprüche 1 bis 8, **dadurch gekennzeichnet, daß** das Textilmaterial ein Gewebe, Gewirke oder Vliesstoff mit einem Flächengewicht von 10 bis 200, vorzugsweise 10 bis 50 g/m$^2$ ist.

10. Konstruktion gemäß mindestens einem der Ansprüche 1 bis 9, **dadurch gekennzeichnet, daß** die Auflage aus einem hydrophilen oder hydrophilierten Material besteht.

11. Konstruktion gemäß mindestens einem der Ansprüche 1 bis 10, **dadurch gekennzeichnet, daß** die Auflage einen hydrophilierungswirksamen Überzug eines N-Acyl-N-alkyl-glycinats der Formel I

$$R\text{-}CO\text{-}N(R^1)\text{-}CH_2\text{-}COOMe \qquad\qquad (I)$$

aufweist,
worin R einen aliphatischen Rest mit 9 bis 23 C-Atomen, insbesondere mit 11 bis 17 C-Atomen bedeutet, der gesättigt ist oder eine bis drei Doppelbindungen aufweist,
R$^1$ Wasserstoff oder Niederalkyl mit 1 bis 4 C-Atomen und
Me ein Wasserstoff- oder Metallatom ist.

**12.** Konstruktion gemäß mindestens einem der Ansprüche 1 bis 11, **dadurch gekennzeichnet, daß** die Auflage Bestandteil eines flexiblen Teststreifens ist.

**13.** Verfahren zur Herstellung einer Konstruktion aus einem flächenförmigen, flexiblen Objekt und einer flächenförmigen funktionellen Auflage für zumindest einen Bereich des flächenförmigen, flexiblen Objekts, wobei die Auflage mit dem Objekt nicht vollflächig verbunden ist, und wobei die Auflage beim Biegen des Verbunds, bestehend aus flexible Objekt und Auflage, nach der Seite, auf der sich die Auflage befindet, keine Falte bildet, **dadurch gekennzeichnet, daß** man ein flächenförmiges Auflageelement, oder mehrere flächenförmige Auflageelemente, die, sich überlappend, gemeinsam die Auflage bilden, auf dem flexiblen Objekt so befestigt, daß ein Teil ihrer Fläche gegenüber der von diesem Teil abgedeckten Objektfläche in Richtung der beim Biegen des Objekts erzeugten Krümmung frei verschiebbar ist.

**14.** Verfahren gemäß Anspruch 13, **dadurch gekennzeichnet, daß** man flächenförmige Auflageelemente einsetzt, die aus einem Textilmaterial mit den in einem der Ansprüche 7 bis 10 angegebenen Merkmalen bestehen.

**15.** Verfahren gemäß mindestens einem der Ansprüche 13 und 14, **dadurch gekennzeichnet, daß** man flächenförmige Auflageelemente einsetzt, die mit einem N-Acyl-N-alkyl-glycinat der Formel I hydrophiliert sind.

**16.** Teststreifen aus einem gegebenenfalls transparenten oder mit Inspektionsöffnungen versehenen, flexiblen, flächenförmigen Träger, auf dem ein oder mehrere Testfelder nebeneinander angeordnet sind, die jeweils eine oder mehrere übereinanderliegende Nachweisschichten tragen, **dadurch gekennzeichnet, daß** die Testfelder durch eine auf ihm lose aufliegende funktionelle Auflage entsprechend einer Konstruktion gemäß mindestens einem der Ansprüche 1 bis 12 abgedeckt sind.

**17.** Teststreifen gemäß Anspruch 16, **dadurch gekennzeichnet, daß** er mindestens zwei Testfelder, die Nachweis-Reagenzien für den gleichen oder unterschiedliche diagnostisch verwertbare Analyte aufweist.

**18.** Teststreifen gemäß mindestens einem der Ansprüche 16 und 17, **dadurch gekennzeichnet, daß** die Testfelder durch die verschiebbaren Flächenbereiche einer zweiteiligen funktionellen Auflage gemäß Anspruch 5 bedeckt sind.

**19.** Teststreifen gemäß mindestens einem der Ansprüche 16 bis 18, **dadurch gekennzeichnet, daß** die Überlappung der beiden Auflageelemente über der Trennungslinie zwischen den beiden Testfeldern und vorzugsweise symmetrisch dazu liegt.

**20.** Teststreifen gemäß mindestens einem der Ansprüche 16 bis 19, **dadurch gekennzeichnet, daß** die Anordnung von Nachweisschichten und Auflagen auf dem Teststreifen mit einem inerten flächenförmigen Material so abgedeckt ist, daß nur im Bereich der Überlappung der Auflageelemente in Richtung der Längsachse des Teststreifens gesehen, eine für den Probenauftrag ausreichende Strecke freibleibt.

**21.** Teststreifen gemäß mindestens einem der Ansprüche 16 bis 20, **dadurch gekennzeichnet, daß** die von der inerten Abdeckung für den Probenauftrag freigelassene Strecke 2 bis 5 mm beträgt.

**22.** Teststreifen gemäß mindestens einem der Ansprüche 16 bis 21, **dadurch gekennzeichnet, daß** die Hydrophilie, die Durchlässigkeit und das Flüssigkeitsleitvermögen des Auflagematerials so abgestimmt sind, daß eine Analyt-Probe über den gesamten analytsensitiven Bereich des Testträgers verteilt wird, der Teststreifen selbstdosierend ist, bzw. überschüssige Probenmenge über dem Auftragspunkt stehen bleibt.

**23.** Verfahren zur Herstellung eines diagnostischen Teststreifens bei dem man auf einem gegebenenfalls transparenten oder mit Inspektionsöffnungen versehenen Träger ein- oder mehrschichtige Nachweisfelder aufbringt, diese mit einer seitlich von den Testfeldern fixierten auf den Testfeldern lose aufliegenden funktionellen Auflage überdeckt und gegebenenfalls noch eine inerte Abdeckung, die nur die Probenauftragsstelle freiläßt aufbringt, **dadurch gekennzeichnet, daß** man ein flächenförmiges Auflageelement, oder mehrere flächenförmige Auflageelementen, die, sich überlappend, gemeinsam die Auflage bilden, auf dem Teststreifen neben dem (den) Testfeld(ern) so befestigt, daß ein Teil ihrer Fläche das (die) Testfeld(er) überdeckt und gegenüber dem (den) Testfeld(ern) in Richtung der beim Biegen des Objekts erzeugten Krümmung frei verschiebbar ist.

**24.** Verfahren gemäß Anspruch 23, **dadurch gekennzeichnet, daß** man flächenförmige Auflageelemente einsetzt, die aus einem Textilmaterial mit den in einem der Ansprüche 7 bis 10 angegebenen Merkmalen bestehen.

25. Verfahren gemäß mindestens einem der Ansprüche 23 und 24, **dadurch gekennzeichnet, daß** man flächenförmige Auflageelemente einsetzt, die mit einem N-Acyl-N-alkyl-glycinat der Formel I hydrophiliert sind.

26. Verwendung eines diagnostischen Teststreifens gemäß einem der Patentansprüche 16 bis 22 zur Bestimmung von Analyt in einer Flüssigkeit.

27. Verfahren zur Bestimmung von Analyt in einer flüssigen Probe, wobei Probenflüssigkeit auf die Probenauftragsstelle aufgegeben wird und die Nachweisschicht(en) auf eine Signalbildung hin beobachtet wird (werden), wobei die Signalbildung ein Maß für die Anwesenheit bzw. Menge an Analyt in der untersuchten flüssigen Probe darstellt, **dadurch gekennzeichnet, daß** ein diagnostischer Teststreifens gemäß einem der Patentansprüche 16 bis 22 eingesetzt wird.

**Claims**

1. Construction comprising a flat-shaped object and a flat-shaped functional overlay for at least one region of the flat-shaped flexible object which is not connected over its whole area with the object and does not form a fold when the sandwich composed of the flexible object and overlay is bent towards the side on which the overlay is located, wherein the overlay is composed of one or more flat-shaped overlay elements which are attached to the flexible object in such a way that a part of their surface overlaps relative to the surface of the object covered by this part in the direction of curvature produced when the object is bent such that the surface can move freely.

2. Construction as claimed in claim 1 wherein the overlay element is attached to the flexible object by at least one, preferably at least two, attachment points which are in a connected surface area (attachment region) of the overlay element which extends between the edges of the overlay element lying in the direction of curvature and whose boundary to the freely movable part or freely movable parts of the overlay element is essentially a straight line and at right angles to the direction of curvature.

3. Construction as claimed in at least one of the claims 1 and 2, wherein the attachment region comprises one end of the overlay element.

4. Construction as claimed in at least one of the claims 1 to 3, wherein the movable end of the overlay overlaps with an overlay having the same or any other type of attachment.

5. Construction as claimed in at least one of the claims 1 to 4, wherein the overlay is composed of two overlay elements whose movable regions face one another and overlap.

6. Construction as claimed in at least one of the claims 1 to 5, wherein the overlay elements overlap over a length of 0.5 to 2.5 mm.

7. Construction as claimed in at least one of the claims 1 to 6, wherein the overlay is composed of an open pore flat-shaped structure made of a textile material which is itself not capillary-active and its construction and the material of the open pore flat-shaped structure and/or its finish are selected such that it spreads 10 µl of water over an area of more than 300 mm$^2$ when resting on a support.

8. Construction as claimed in at least one of the claims 1 to 7, wherein the textile material has a thickness of 20 to 200, in particular 30 to 100 µm and/or a pore volume of 30 to 85, in particular 40 to 75 %.

9. Construction as claimed in at least one of the claims 1 to 8, wherein the textile material is a fabric, knitted fabric or fleece material with a weight per unit area of 10 to 200, preferably 10 to 50 g/m$^2$.

10. Construction as claimed in at least one of the claims 1 to 9, wherein the overlay is composed of a hydrophilic or hydrophilized material.

11. Construction as claimed in at least one of the claims 1 to 10, wherein the overlay has a hydrophilizing coating of an N-acyl-N-alkyl-glycinate of formula I

$$R\text{-}CO\text{-}N(R^1)\text{-}CH_2\text{-}COOMe \hspace{3cm} (I)$$

in which R denotes an aliphatic residue with 9 to 23 C atoms, in particular with 11 to 17 C atoms, which is saturated or has one to three double bonds,
$R^1$ is hydrogen or lower alkyl with 1 to 4 C atoms and
Me is a hydrogen or metal atom.

12. Construction as claimed in at least one of the claims 1 to 11, wherein the overlay is a component of a flexible test strip.

13. Process for the production of a construction consisting of a flat-shaped, flexible object and a flat-shaped functional overlay for at least one region of the flat-shaped flexible object, where said overlay is not attached with its whole area to the object and where said overlay does not form a fold when the sandwich composed of the flexible object and overlay is bent towards the side on which the overlay is located, wherein a flat-shaped overlay element or several overlapping flat-shaped overlay elements that together form the overlay are attached to the flexible object in such a way that a part of its/their surface can move freely relative to the area of the object covered by this part in the direction of curvature created by bending the object.

14. Process as claimed in claim 13, wherein flat-shaped overlay elements are used which are composed of a textile material with the features stated in one of the claims 7 to 10.

15. Process as claimed in at least one of the claims 13 and 14, wherein flat-shaped overlay elements are used which are hydrophilized with an N-acyl-N-alkylglycinate of formula I.

16. Test strip composed of a flexible flat-shaped support that is optionally transparent or provided with inspection openings, on which one or more test fields are arranged next to one another which each carry one or more detection layers stacked on top of one another, wherein the test fields are covered by a functional overlay corresponding to a construction as claimed in at least one of the claims 1 to 12 which rests loosely on them.

17. Test strip as claimed in claim 16, wherein it has at least two test fields which contain detection reagents for the same or different analytes that can be used diagnostically.

18. Test strip as claimed in at least one of the claims 16 and 17, wherein the test fields are covered by the movable zones of a two-part functional overlay as claimed in claim 5.

19. Test strip as claimed in at least one of the claims 16 to 18, wherein the overlap of the two overlay elements is above the separation line between the two test fields and preferably symmetrical thereto.

20. Test strip as claimed in at least one of the claims 16 to 19, wherein the arrangement of detection layers and overlays on the test strip is covered with an inert flat-shaped material in such a manner that a section only remains free that is adequate for sample application in the overlap region of the overlay elements viewed in the direction of the longitudinal axis of the test strip.

21. Test strip as claimed in at least one of the claims 16 to 20, wherein the section left free by the inert cover for sample application is 2 to 5 mm.

22. Test strip as claimed in at least one of the claims 16 to 21, wherein the hydrophilicity, transparency and the liquid conducting capacity of the overlay material are matched in such a manner that an analyte sample is distributed over the entire analyte-sensitive region of the test carrier, the test strip is self-dosing and excess sample remains above the application spot.

23. Process for the production of a diagnostic test strip in which single or multilayer detection fields are mounted on a support which is optionally transparent or provided with inspection openings, these are covered with a functional overlay which rests loosely on the test fields and is attached at the sides of the test fields and optionally an inert cover which only leaves the sample application site free is additionally mounted, wherein a flat-shaped overlay element or several overlapping flat-shaped overlay elements that together form the overlay are attached to the test strip next to the test field(s) in such a way that a part of their surface covers the test field(s) and can move freely relative to the test field(s) in the direction of curvature produced when the object is bent.

24. Process as claimed in claim 23, wherein flat-shaped overlay elements are used which are composed of a textile material with the features stated in one of the claims 7 to 10.

25. Process as claimed in at least one of the claims 23 and 24, wherein flat-shaped overlay elements are used which are hydrophilized with an N-acyl-N-alkylglycinate of formula I.

26. Use of a diagnostic test strip as claimed in one of the claims 16 to 22 to determine an analyte in a liquid.

27. Method for the determination of an analyte in a liquid sample, in which a sample liquid is applied to the sample application site and the detection layer(s) is(are) observed for signal generation, the signal generation being a measure for the presence or the amount of analyte in the examined liquid sample, wherein a diagnostic test strip as claimed in one of the claims 16 to 22 is used.

**Revendications**

1. Construction comprenant un objet plat, souple, et un revêtement fonctionnel plat pour au moins une zone de l'objet plat, souple, qui n'est pas relié sur toute sa surface avec l'objet et qui ne forme pas de plis lors de la flexion du composite, constitué par l'objet souple et le revêtement, vers le côté sur lequel se trouve le revêtement, **caractérisé en ce que** le revêtement est constitué par un ou plusieurs éléments de revêtement plats, qui sont fixés sur l'objet souple de telle manière qu'une partie de leur surface est disposée avec un recouvrement par rapport à la surface de l'objet recouverte par cette partie dans le sens de la courbure obtenue lors de la flexion de l'objet afin que la surface puisse se déplacer librement.

2. Construction selon la revendication 1, **caractérisée en ce que** la fixation de l'élément de revêtement sur l'objet souple est réalisée par au moins un, de préférence au moins deux points de fixation, qui se trouvent dans une zone cohérente de la surface (zone de fixation) de l'élément de revêtement, qui s'étend entre les bords de l'élément de revêtement se trouvant dans le sens de la courbure et dont la délimitation par rapport à la ou les parties qui peuvent se déplacer librement de l'élément de revêtement s'étend de manière essentiellement linéaire et transversale par rapport au sens de courbure.

3. Construction selon au moins l'une quelconque des revendications 1 et 2, **caractérisée en ce que** la zone de fixation comprend une extrémité de l'élément de revêtement.

4. Construction selon au moins l'une quelconque des revendications 1 à 3, **caractérisée en ce que** l'extrémité mobile du revêtement recouvre un revêtement présentant un type de fixation identique ou différent.

5. Construction selon au moins l'une quelconque des revendications 1 à 4, **caractérisée en ce que** le revêtement est constitué par deux éléments de revêtement, dont les zones mobiles sont orientées l'une contre l'autre et se recouvrent.

6. Construction selon au moins l'une quelconque des revendications 1 à 5, **caractérisée en ce que** les éléments de revêtement se recouvrent sur une longueur de 0,5 à 2,5 mm.

7. Construction selon au moins l'une quelconque des revendications 1 à 6, **caractérisée en ce que** le revêtement est constitué par une structure plane à pores ouverts en un matériau textile qui n'est pas actif en capillarité et **en ce que** la construction et le matériau de la structure plane à pores ouverts et/ou son apprêt est choisi de telle manière qu'il étale, en se trouvant sur un substrat, 10 µl d'eau sur une surface supérieure à 300 mm$^2$.

8. Construction selon au moins l'une quelconque des revendications 1 à 7, **caractérisée en ce que** le matériau textile présente une épaisseur de 20 à 200, en particulier de 30 à 100 µm et/ou un volume de pores de 30 à 85, en particulier de 40 à 75%.

9. Construction selon au moins l'une quelconque des revendications 1 à 8, **caractérisée en ce que** le matériau textile est un tissu, un tricot ou un non-tissé, présentant un poids surfacique de 10 à 200, de préférence de 10 à 50 g/m$^2$.

**10.** Construction selon au moins l'une quelconque des revendications 1 à 9, **caractérisée en ce que** le revêtement est constitué par un matériau hydrophile ou hydrophilisé.

**11.** Construction selon au moins l'une quelconque des revendications 1 à 10, **caractérisée en ce que** le revêtement présente un recouvrement actif en hydrophilisation en un
N-acyl-N-alkyl-glycinate de formule I

$$R\text{-}CO\text{-}N(R^1)\text{-}CH_2\text{-}COOMe \qquad\qquad (I)$$

dans laquelle R signifie un radical aliphatique comprenant 9 à 23 atomes de carbone, en particulier 11 à 17 atomes de carbone, qui est saturé ou qui présente une à trois doubles liaisons,
$R^1$ représente hydrogène ou alkyle inférieur, comprenant 1 à 4 atomes de carbone et
Me représente un atome d'hydrogène ou de métal.

**12.** Construction selon au moins l'une quelconque des revendications 1 à 11, **caractérisée en ce que** le revêtement est un constituant d'une bandelette test souple.

**13.** Procédé de fabrication d'une construction constituée par un objet plat, souple et un revêtement plat fonctionnel pour au moins une zone de l'objet plat, souple, le revêtement n'étant pas complètement relié avec toute la surface de l'objet et le revêtement ne formant pas de plis lors de la flexion du composite, constitué par l'objet souple et le revêtement, vers le côté sur lequel se trouve le revêtement, **caractérisé en ce qu'**on fixe un élément de revêtement plat ou plusieurs éléments de revêtement plats qui forment ensemble, en se recouvrant, le revêtement, sur l'objet souple de telle manière qu'une partie de leur surface peut se déplacer librement par rapport à la surface de l'objet recouverte par cette partie dans le sens de la courbure obtenue lors de la flexion de l'objet.

**14.** Procédé selon la revendication 13, **caractérisé en ce qu'**on utilise des éléments de revêtement plats qui sont constitués par un matériau textile présentant les caractéristiques indiquées dans l'une quelconque des revendications 7 à 10.

**15.** Procédé selon au moins l'une quelconque des revendications 13 et 14, **caractérisé en ce qu'**on utilise des éléments de revêtement plats qui sont hydrophilisés avec un N-acyl-N-alkyl-glycinate de formule I.

**16.** Bandelette test constituée par un support souple plat, le cas échéant transparent ou pourvu d'ouverture d'inspection, sur lequel sont disposés un ou plusieurs champs test l'un à côté de l'autre, qui présentent à chaque fois une ou plusieurs couches de détection superposées, **caractérisée en ce que** les champs test sont recouverts par un revêtement fonctionnel placé librement sur ceux-ci de manière correspondante à une construction selon au moins l'une quelconque des revendications 1 à 12.

**17.** Bandelette test selon la revendication 16, **caractérisée en ce qu'**elle présente au moins deux champs test, qui présentent des réactifs de détection pour des analytes identiques ou différents utilisables en diagnostic.

**18.** Bandelette test selon au moins l'une quelconque des revendications 16 et 17, **caractérisée en ce que** les champs test sont recouverts par les zones planes pouvant être déplacées d'un revêtement fonctionnel en deux parties selon la revendication 5.

**19.** Bandelette test selon au moins l'une quelconque des revendications 16 à 18, **caractérisée en ce que** le recouvrement des deux éléments de revêtement se place au-dessus de la ligne de séparation entre les deux champs test et est de préférence symétrique par rapport à celle-ci.

**20.** Bandelette test selon au moins l'une quelconque des revendications 16 à 19, **caractérisée en ce que** la disposition des couches de détection et des revêtements sur la bandelette test est recouverte par un matériau plat inerte de telle manière qu'il ne reste une zone libre suffisante pour l'application d'un échantillon qu'au niveau de la zone du recouvrement des éléments de revêtement dans le sens de l'axe longitudinal de la bandelette test.

**21.** Bandelette test selon au moins l'une quelconque des revendications 16 à 20, **caractérisée en ce que** la zone laissée libre par le recouvrement inerte pour l'application d'un échantillon est de 2 à 5 mm.

**22.** Bandelette test selon au moins l'une quelconque des revendications 16 à 21, **caractérisée en ce que** l'hydrophilie, la perméabilité et le pouvoir de guidage d'un liquide du matériau de revêtement sont adaptés de telle manière qu'un échantillon avec des analytes est réparti sur toute la zone sensible aux analytes du support test, la bandelette test est auto-dosante, resp. la quantité d'échantillon en excès reste au-dessus du point d'application.

**23.** Procédé pour la fabrication d'une bandelette test diagnostique dans lequel on applique sur un support le cas échéant transparent ou pourvu d'ouvertures d'inspection, des champs de détection à une ou plusieurs couches, on recouvre ceux-ci par un revêtement fonctionnel disposé librement sur les champs test, fixé latéralement par rapport aux champs test et on applique le cas échéant encore un recouvrement inerte, qui ne laisse libre que le site d'application de l'échantillon, **caractérisé en ce qu'**on fixe un élément de revêtement plat ou plusieurs éléments de revêtement plats qui forment ensemble, en se recouvrant, le revêtement, sur la bandelette test, à côté du ou des champs test, de telle manière qu'une partie de leur surface recouvre le ou les champs test et qu'il peut être déplacé librement par rapport au(x) champ(s) test dans le sens de la courbure obtenue lors de la flexion de l'objet.

**24.** Procédé selon la revendication 23, **caractérisé en ce qu'**on utilise des éléments de revêtement plats, qui sont constitués par un matériau textile présentant les caractéristiques indiquées dans l'une quelconque des revendications 7 à 10.

**25.** Procédé selon au moins l'une quelconque des revendications 23 et 24, **caractérisé en ce qu'**on utilise des éléments de revêtement plats qui sont hydrophilisés avec un N-acyl-N-alkyl-glycinate de formule I.

**26.** Utilisation d'une bandelette test diagnostique selon l'une quelconque des revendications 16 à 22 pour la détermination d'un analyte dans un liquide.

**27.** Procédé pour la détermination d'un analyte dans un échantillon liquide, dans lequel le liquide de l'échantillon est appliqué sur le site d'application d'un échantillon et on observe la formation d'un signal sur la ou les couches de détection, la formation du signal étant une mesure de la présence resp. de la quantité d'analyte dans l'échantillon liquide analysé, **caractérisé en ce qu'**on utilise une bandelette test diagnostique selon l'une quelconque des revendications 16 à 22.

Fig. 1

Fig. 2

Fig. 3

Fig.4

Fig.5

Fig.6

Fig.7

Fig. 8

Fig. 9

Fig. 10

Fig.11

Fig.12

Fig. 13

Fig. 14

30